(19) Europäisches Patentamt
European Patent Office
Office européen des brevets

(11) **EP 3 733 062 A1**

(12) **EUROPEAN PATENT APPLICATION**
published in accordance with Art. 153(4) EPC

(43) Date of publication:
**04.11.2020 Bulletin 2020/45**

(51) Int Cl.:
***A61B 5/05*** *(2006.01)*

(21) Application number: **18897720.1**

(22) Date of filing: **28.12.2018**

(86) International application number:
**PCT/JP2018/048511**

(87) International publication number:
**WO 2019/132013 (04.07.2019 Gazette 2019/27)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.12.2017 JP 2017254712**

(71) Applicant: **Tanita Corporation
Tokyo 174-8630 (JP)**

(72) Inventors:
• **NAGAHAMA, Toshiki
Tokyo 174-8630 (JP)**
• **TAKEHARA, Tomoko
Tokyo 174-8630 (JP)**
• **KASAHARA, Yasuhiro
Tokyo 174-8630 (JP)**

(74) Representative: **Barker Brettell LLP
100 Hagley Road
Edgbaston
Birmingham B16 8QQ (GB)**

(54) **CONDITION EVALUATION DEVICE, CONDITION EVALUATION METHOD, AND PROGRAM**

(57) A biometric device 10 serving as a condition evaluation device includes a body weight measurement unit 102 configured to acquire a body weight of a user, an electric resistance measurement unit 103 configured to acquire a bioimpedance of a specific part in the user, and a condition evaluation unit 105 configured to evaluate a condition of the specific part based on the body weight of the user and the bioimpedance of the specific part.

FIG.2

EP 3 733 062 A1

**Description**

TECHNICAL FIELD

The present invention relates to a condition evaluation device, a condition evaluation method, and a program configured to evaluate a condition of a body.

BACKGROUND ART

Generally, when inflammation of a body part or the like occurs, a body water content increases in the part, and thus a bioimpedance of the part decreases. As a determination device that utilizes such a phenomenon, a device has been proposed that measures a bioimpedance of a user, and determines a condition at an affected part of the user by using the value measured (see JP4422997B).

SUMMARY OF INVENTION

However, the bioimpedance of the user varies within a day and also varies over days. In the device described above, such a diurnal variation and interday variation in the bioimpedance might offset a change in the bioimpedance due to an occurrence of inflammation or the like. This may result in the body part of the user erroneously being determined to be normal.
The present invention is made in view of such a problem, and an object of the present invention is to provide a condition evaluation device, a condition evaluation method, and a program configured to accurately evaluate a condition at a body part of a user.
According to an aspect of the present invention, a condition evaluation device includes a body weight acquisition unit configured to acquire a body weight of a user, an impedance acquisition unit configured to acquire a bioimpedance of a specific part in the user, and an evaluation unit configured to evaluate a condition of the specific part based on the body weight of the user and the bioimpedance of the specific part.
According to this aspect, the condition of a body part in the user is evaluated while taking into consideration not only the bioimpedance of the user but also the body weight of the user correlated with the diurnal and interday variation in the bioimpedance of the user.
With the body weight of the user also taken into consideration, identification of a variation component due to the diurnal and interday variation in an acquired value of the bioimpedance of the user is facilitated. Thus, the variation component of the bioimpedance due to a change in the condition of the user's body part can be accurately extracted. Accordingly, the condition of the user's body part can be accurately evaluated.

BRIEF DESCRIPTION OF DRAWINGS

[Fig. 1] Fig. 1 is a schematic view illustrating an example of an outer appearance of a biometric device according to a first embodiment in the present invention.
[Fig. 2] Fig. 2 is a block diagram illustrating an example of a functional configuration in the biometric device.
[Fig. 3] Fig. 3 is a circuit diagram showing an example of a configuration of an equivalent circuit related to a body cell.
[Fig. 4] Fig. 4 is a diagram illustrating relationship between a reactance component and a resistance component due to a change in the frequency of alternating electric current applied to a user.
[Fig. 5] Fig. 5 is a diagram illustrating relationship between a change in an electric resistance of the extracellular fluid and a change in the minimum point of the reactance component.
[Fig. 6] Fig. 6 is a diagram illustrating a relationship between the resistance component of a bioimpedance and a body weight.
[Fig. 7] Fig. 7 is a flowchart illustrating an example of a process procedure of a condition evaluation method according to the first embodiment.
[Fig. 8] Fig. 8 is a flowchart illustrating an example of a process procedure of a condition evaluation process.
[Fig. 9] Fig. 9 is a diagram illustrating an example of a method for correcting an inflammation level according to a second embodiment.
[Fig. 10A] Fig. 10A is a diagram illustrating an example of relationship between a resistance component and a body weight of a user in a case where a determination result indicates normal.
[Fig. 10B] Fig. 10B is a diagram illustrating an example of a checking method in the case where the determination result indicates normal according to a third embodiment.

[Fig. 11A] Fig. 11A is a diagram illustrating a relationship between a resistance component and a body weight of a user in a case where a determination result indicates normal.

[Fig. 11B] Fig. 11B is a diagram illustrating an example of a checking method in the case where the determination result indicates normal according to a fourth embodiment.

[Fig. 12] Fig. 12 is a diagram illustrating an example of a method for detecting excessive reduction of a body water content of a user according to the fifth embodiment.

[Fig. 13] Fig. 13 is a flowchart illustrating an example of a process procedure of a condition determination process according to the fifth embodiment.

[Fig. 14] Fig. 14 is a diagram illustrating an example of a method for detecting a long-term dehydration according to a sixth embodiment.

[Fig. 15] Fig. 15 is a flowchart indicating a process procedure of a condition evaluation method according to the sixth embodiment.

[Fig. 16A] Fig. 16A is a diagram illustrating an example of a method for detecting a muscle developed condition of a measurement part according to a seventh embodiment.

[Fig. 16B] Fig. 16B is a diagram illustrating an example of a method for detecting a muscle atrophied condition of a measurement part according to the seventh embodiment.

DESCRIPTION OF EMBODIMENTS

Embodiment of the present invention will be described below with reference to the attached drawings.

(First embodiment)

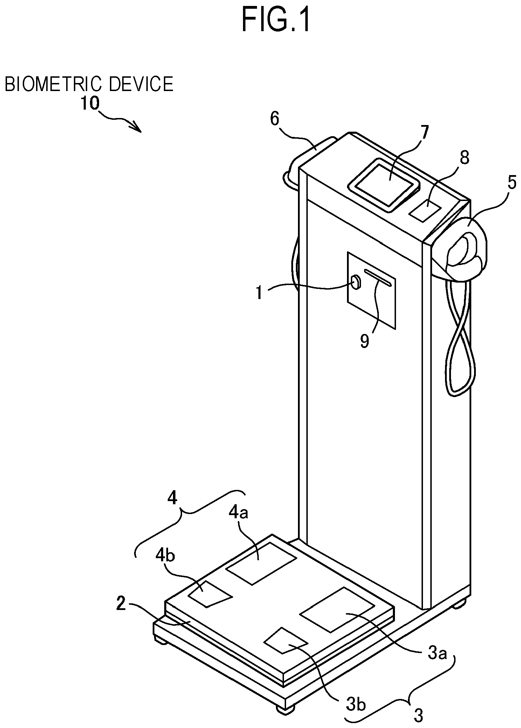

Fig. 1 is a schematic view illustrating an example of an outer appearance of a biometric device 10 according to a first embodiment of the present invention.

The biometric device 10 serves as a condition evaluation device configured to evaluate a condition of a user. The biometric device 10 includes a power switch 1 configured to supply power to the biometric device 10, a body scale 2 configured to measure the body weight of the user, and an electrode portion 3 and an electrode portion 4 configured to measure the bioimpedance of both user's legs. The biometric device 10 further includes an electrode portion 5 and an electrode portion 6 configured to measure the biometric impedance of both user's arms, a display operation device 7, a communication device 8, and a printer 9.

The body scale 2 has, on a board on which the user stands on his or her feet, the electrode portion 3 arranged to be in contact with the right foot of the user and with the electrode portion 4 arranged to be in contact with the left foot of the user.

The electrode portion 3 includes an electrode 3a adapted to make electric current flow in the right foot of the user, and an electrode 3b adapted to detect voltage produced in the right foot due to the application of the electric current to the right foot from the electrode 3a. The electrode portion 4 includes an electrode 4a adapted to make electric current flow in the left foot of the user, and an electrode 4b adapted to detect voltage produced in the left foot due to the application of the electric current to the left foot from the electrode 4a.

The electrode portion 5 includes a first electrode adapted to make electric current flow in the right hand of the user and a second electrode adapted to detect voltage produced in the right hand due to the application of the electric current to the right hand from the first electrode. The electrode portion 6 includes a first electrode adapted to make electric current flow in the left hand of the user and a second electrode adapted to detect voltage produced in the left hand due to the application of the electric current to the left hand from the first electrode.

The display operation device 7 receives basic biometric information set by an input operation by a user, and displays an evaluation result of the condition of entire body or a body part in the user. Examples of the basic biometric information include the height, age, and gender of the user. For example, the display operation device 7 is implemented by a touch panel type or push button type liquid crystal display device.

The communication device 8 communicates with an external terminal (not shown). For example, the communication device 8 communicates with the external terminal through near field communications or a mobile phone network. The communication device 8 according to the present embodiment transmits the biometric information obtained by the biometric device 10 to the external terminal, and receives biometric data including past measurement values related to the user and the like from the external terminal.

In this manner, the biometric device 10 according to the present embodiment measures the body weight of the user and measures the bioimpedance of the user. The bioimpedance of the user is measured with the user holding the electrode portion 5 with the right hand, holding the electrode portion 6 with the left hand, stepping on the electrode portion 3 with the right foot, and stepping on the electrode portion 4 with the left foot. In this state, the biometric device 10 measures the bioimpedance of each part in the user, that is, the right leg, the left leg, the right arm, and the left arm.

As the biometric device 10, a biometric device may be used that has band type electrode portions to be placed around both hands and both feet, instead of or in addition to the electrode portions 3 to 6. When the band type electrode portions are used, the user can select the measurement parts as desired. The electrode portion does not necessarily be of a band type, and any electrode portion enabling the measurement part to be selected as desired may be used. For example. a clip type electrode portion and the like may be used.

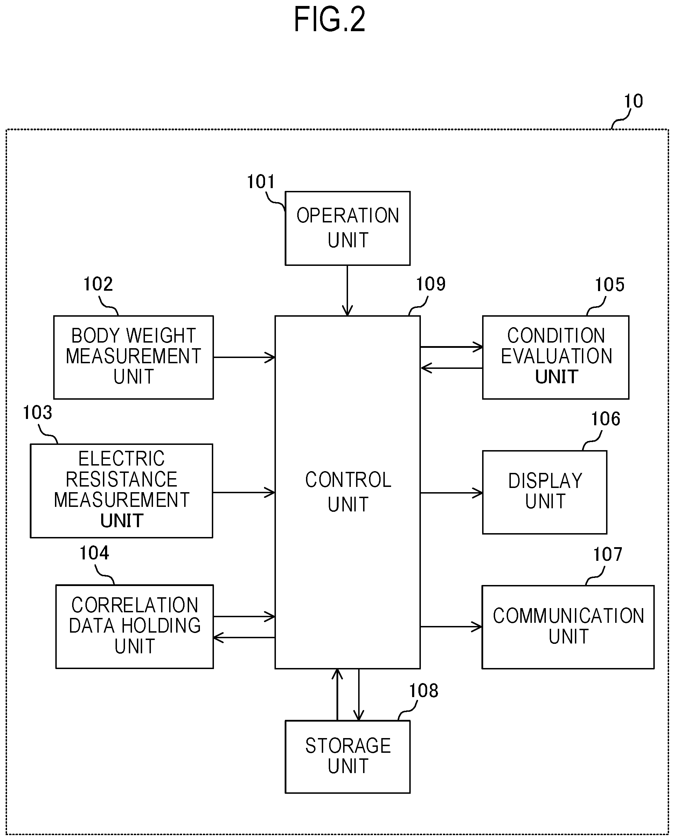

Fig. 2 is a block diagram illustrating a main functional configuration of the biometric device 10 according to the present embodiment.

The biometric device 10 includes an operation unit 101, a body weight measurement unit 102, an electric resistance measurement unit 103, a correlation data holding unit 104, a condition evaluation unit 105, a display unit 106, a communication unit 107, a storage unit 108, and a control unit 109.

The operation unit 101 receives an operation of switching the power switch 1 shown in Fig. 1 ON or OFF. The operation unit 101 receives basic biometric information about the user by an operation of the user. For example, information set by a touch sensor, a button, a dial, or the like is input to the operation unit 101.

The body weight measurement unit 102 serves as a body weight acquisition unit configured to acquire the body weight of the user. For example, the body weight acquisition unit includes a measurement instrument such as the body scale 2 that measures the body weight of the user, a measurement device that measures the body weight of the user on the basis of a value measured by the measurement instruments as the body weight of the user, or a device that receives and acquires body weight data indicating the body weight of the user from an external measurement device or an external terminal.

The body weight measurement unit 102 according to the present embodiment measures the body weight of the user by using the body scale 2 shown in Fig. 1. For example, the body weight measurement unit 102 may estimate the weight of the cloths based on the basic biometric information, and calculate a value obtained by subtracting this estimated weight from the measurement value of the body scale 2, as the body weight of the user.

The electric resistance measurement unit 103 serves as an impedance acquisition unit that acquires the bioimpedance related to a specific part of the user. This specific part may be any part that can be measured using a measurement instrument, and may be body parts such as the right leg, the left leg, the right arm, and the left arm for example.

For example, the impedance acquisition unit described above includes an electric resistance measurement instrument that measures the impedance of the specific part in the user, or a measurement device that measures the impedance of the specific part in the user on the basis of a value measured by the electric resistance measurement instrument as the impedance of the specific part in the user. The impedance acquisition unit may receive and acquire the electric resistance data indicating the bioimpedance of the specific part in the user from an external measurement device or an external terminal.

The electric resistance measurement unit 103 according to the present embodiment uses the electrode portions 3 to 6 shown in Fig. 1 to apply alternating electric current at a specific frequency to the parts of the user, and detects the alternating voltage of the parts with the alternating electric current applied. The electric resistance measurement unit 103 uses the applied electric current value and the detected voltage value of the parts in the user, to measure the bioimpedance of the entire body and the parts in the user.

For example. the electric resistance measurement unit 103 may calculate at least one of a resistance component and an inductance component of the bioimpedance for each measurement part. Such an electric resistance measurement unit 103 serves as a component calculation unit configured to calculate the values of the reactance component and the resistance component of the bioimpedance.

Furthermore, the electric resistance measurement unit 103 may apply the basic biometric information (such as the height, age, and gender) that set by the user on the operation unit 101, to a predetermined regression equation to calculate other biometric indicators. Examples of the other biometric indicators include fat percentage, fat mass, lean mass, muscle mass, visceral fat mass, visceral fat level, visceral fat area, subcutaneous fat mass, basal metabolic rate, bone mass, body water percentage, body water content, Body Mass Index (BMI), intracellular fluid volume, extracellular fluid volume, and the like of the entire body and each part.

The correlation data holding unit 104 holds, for each body part, correlation data indicating the correlation between the bioimpedance and the body weight of a person, for identifying diurnal variation and interday variation in the bioimpedance of the user. The correlation data held by the correlation data holding unit 104 is acquired from the storage unit 108 in response to an instruction from the control unit 109 when the power switch 1 is turned ON, for example.

Examples of the correlation data described above include coefficients of a function representing a predetermined regression line indicating the relationship between the body weight in the entire body of the user and the bioimpedance at a specific body part of the user, and personal data or group data of the user(s) for obtaining the regression line. The personal data is time series data on measurement values of the bioimpedance and the body weight measured for a user in a healthy state. The group data is measurement data as a result of gathering multiple measurement values of the bioimpedance and the body weights of a plurality of persons. The regression line may be a straight line represented by a quadratic function or a curved line represented by a polynomial.

As described above, the correlation data holding unit 104 serves as a regression line acquisition unit that acquires a predetermined regression line representing a correlation between the body weight and the bioimpedance of a body part in a person. The correlation data holding unit 104 according to the present embodiment holds a coefficient of a regression line representing a correlation of the body weight of the user and the bioimpedance of the measurement part, for each of the specific body parts. For example, the correlation data holding unit 104 includes a volatile memory (random access memory (RAM)).

The condition evaluation unit 105 serves as an evaluation unit that evaluates the condition of a specific body part in user on the basis of the body weight of the user and the bioimpedance of the specific body part. The correlation data holding unit 104 is included in this evaluation unit. The condition of the specific body part evaluated by the condition evaluation unit 105 includes a condition involving a change in a body water content in the body part due to an abnormality occurring in the body part.

An example of the condition involving a change in the body water content of a body part includes an edema. A condition evaluation device that evaluates whether a specific body part of the user is inflamed is described below as an embodiment of the condition evaluation device. The condition evaluation device according to the present embodiment evaluates a state where the body water content has increased in a specific body part due to an occurrence of the inflammation in the body part, that is, an occurrence of an edema.

For example, the condition evaluation unit 105 calculates a reference value for the bioimpedance of the measurement part measured by the electric resistance measurement unit 103, on the basis of the measurement value of the body weight of the user. The reference value as used herein is a value taking into consideration a variation in the bioimpedance due to a diurnal variation and interday variation in the body water content.

Specifically, the condition evaluation unit 105 refers to the correlation data holding unit 104 and uses the correlation data to calculate a regression line for each measurement part of the user. The condition evaluation unit 105 calculates, as the reference value, a value of the bioimpedance at each measurement part corresponding to the measurement value of the body weight of the user on the regression line.

The condition evaluation unit 105 compares the measurement value of the bioimpedance with the reference value for each measurement part. The condition evaluation unit 105 determines that the measurement part is inflamed when the measurement value of the bioimpedance is smaller than the reference value, because such a value may be regarded as an indication of an excessive increase in the body water content in the measurement part.

Furthermore, the condition evaluation unit 105 obtains a difference amount between the measurement value of the bioimpedance at a measurement part and the reference value, and calculates an inflammation level indicating severity of the inflammation at the measurement part on the basis of the difference amount. In the present embodiment, the condition evaluation unit 105 obtains a distance from a predefined regression line to a measurement point determined by an acquired value of the body weight of the user and an acquired value of the bioimpedance at the measurement part, and calculates the inflammation level of the measurement part on the basis of the distance. The measurement point as used herein is a coordinate point plotted on a coordinate system with one axis representing the body weight and the other axis representing the bioimpedance of the measurement part. The inflammation level is an example of a body water content change level indicating a level of change in the body water content in the measurement part.

For example, the condition evaluation unit 105 calculates the inflammation level as described above when the measurement part of the user is determined to be inflamed. Generally, a reduction amount of the measurement value with respect to the reference value of the bioimpedance increases as the level of inflammation at the measurement part increases. Thus, in the present embodiment, a larger reduction amount of the measurement value results in a higher inflammation level of the measurement part calculated.

Thus, the condition evaluation unit 105 serves as a calculation unit that calculates the inflammation level of the measurement part by using a predetermined regression line obtained on the basis of the correlation data and by using the measurement values of both bioimpedance and body weight of the user.

For each measurement part of the user, the condition evaluation unit 105 records a determination result indicating whether the measurement part is inflamed, and evaluation data indicating the inflammation level of the measurement part, in the correlation data holding unit 104.

The display unit 106 includes the display operation device 7 shown in Fig. 1. The display unit 106 displays the evaluation data or the determination result stored in the correlation data holding unit 104. The display unit 106 may display the biometric information calculated by the electric resistance measurement unit 103.

The communication unit 107 includes the communication device 8 shown in Fig. 1. For example, the communication unit 107 notifies an external terminal of the user's health condition, by transmitting the body weight measurement value of the user, the measurement value of the bioimpedance at each part of the user, the determination result indicating whether the part is inflamed, the inflammation level, and the like to the external terminal.

The communication unit 107 may receive the correlation data, recorded in the correlation data holding unit 104, from an external terminal. Alternatively, the communication unit 107 may receive time series data indicating the body weight measurement value of the user and the measurement value of the bioimpedance at each part, from an external terminal for generating the correlation data.

The storage unit 108 includes a non-volatile memory (read only memory; ROM), a volatile memory (random access memory; RAM), and the like. The storage unit 108 stores a control program configured to control an operation of the biometric device 10. Thus, the storage unit 108 is a non-transitory computer readable recording medium configured to record a program for executing the functions according to the present embodiment. The storage unit 108 further stores the correlation data.

The control unit 109 includes a central calculation processing device (central processing unit; CPU) serving as a processor, an input interface, and a bus connecting these elements to each other. The control unit 109 reads the control program stored in the storage unit 108 and causes the central calculation processing device to execute the program, to control the components of the biometric device 10 via an input interface.

As described above, the control unit 109 controls each of the operation unit 101, the body weight measurement unit 102, the electric resistance measurement unit 103, the correlation data holding unit 104, the condition evaluation unit 105, the display unit 106, the communication unit 107, and the storage unit 108.

The central calculation processing device forming the control unit 109 may execute the functions of the components such as the operation unit 101, the body weight measurement unit 102, the electric resistance measurement unit 103, the correlation data holding unit 104, the condition evaluation unit 105, the display unit 106, the communication unit 107, and the storage unit 108.

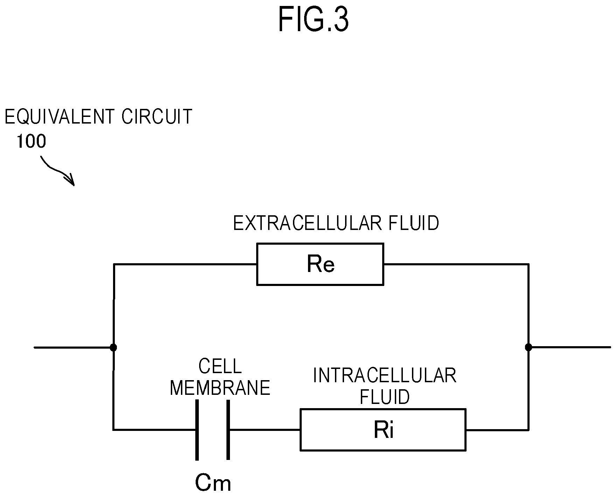

Next, a method for evaluating the level of inflammation at a measurement part according to the present embodiment will be described with reference to Fig. 3 to Fig. 6.

Fig. 3 is a circuit diagram showing an example of a configuration of an equivalent circuit 100 equivalently representing a body cell configuration using electric elements.

The equivalent circuit 100 represents electric characteristics of a biological tissue. In the equivalent circuit 100, a resistance component Re [Ω] corresponds to the electric resistance of an extracellular fluid, a resistance component Ri [Ω] corresponds to the electric resistance of an intercellular fluid, and a capacitance component Cm [μF] corresponds to a capacitance of a cell membrane.

The electric resistance Re of the extracellular fluid largely reflects an increase/reduction of the body water content, and thus is a resistance component that is likely to change within a short period of time. On the other hand, the electric resistance Ri of the intercellular fluid largely reflects the body muscle cell development and thus is a resistance component that is likely to change over a long period of time.

In the equivalent circuit 100, the electric resistance Re of the extracellular fluid is connected in parallel with the capacitance Cm of the cell membrane and the electric resistance Ri of the intercellular fluid connected in series. The following Formula (1) represents an impedance Z of the equivalent circuit 100 corresponding to the bioimpedance.

[Formula 1]

$$Z = \frac{1}{(R_e + R_i)^2 + \left(\dfrac{1}{\omega \cdot C_m}\right)^2} R_e \left\{ R_i (R_e + R_i) + \left(\frac{1}{\omega \cdot C_m}\right)^2 - j \frac{R_e}{\omega \cdot C_m} \right\} \qquad (1),$$

where j represents an imaginary number ($j^2 = -1$).

As indicated in Formula (1), the bioimpedance Z is a complex impedance, and a resistance component R and a reactance component X that are respectively real and imaginary parts of the bioimpedance Z are expressed by the following Formula (2).

[Formula 2]

$$R = \frac{1}{\left(R_e + R_i\right)^2 + \left(\dfrac{1}{\omega \cdot C_m}\right)^2} R_e \left\{ R_i \left(R_e + R_i\right) + \left(\frac{1}{\omega \cdot C_m}\right)^2 \right\}$$

$$X = \frac{1}{\left(R_e + R_i\right)^2 + \left(\dfrac{1}{\omega \cdot C_m}\right)^2} \cdot \frac{-R_e^{\,2}}{\omega \cdot C_m}$$

$$(2)$$

The resistance component R corresponds to the electric resistance of the equivalent circuit 100, and the reactance component X corresponds to the capacitance and the inductance of the equivalent circuit 100. The reactance component X of a positive value corresponds to the inductance component in the equivalent circuit 100. On the other hand, the reactance component X of a negative value corresponds to the capacitance component in the equivalent circuit 100.

As methods to evaluate a condition of the body part of the user using the equivalent circuit 100 shown in Fig. 3, there are a method for capturing a change in the electric resistance Re of the extracellular fluid to detect a short term change in the body water content, and a method for capturing a change in the electric resistance Ri of the intercellular fluid to detect a long term change in the body water content. Of these methods, the method for capturing a change in the electric resistance Re of the extracellular fluid is preferably employed to evaluate the level of inflammation as the internal condition of the measurement part.

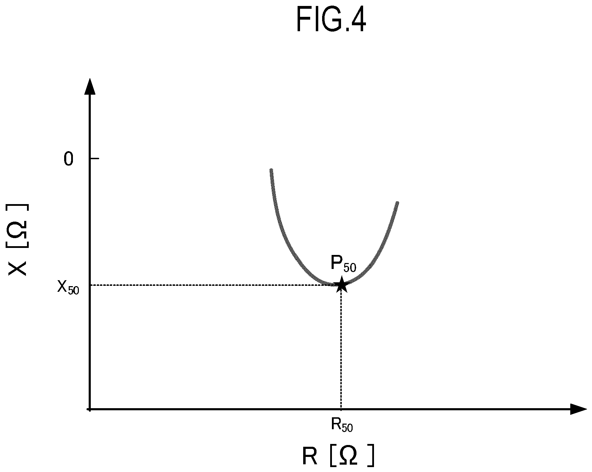

Next, the method for capturing a change in the electric resistance Re of the extracellular fluid will be briefly described with reference to Fig. 4.

Fig. 4 is a diagram illustrating a change in the reactance component X and the resistance component R due to a change in the frequency of the alternating electric current applied to the user. Fig. 4 shows an example of a Cole-Cole plot generated by an alternating impedance method.

The Cole-Cole plot, which is commonly used for a method of expression in the field of electrochemistry, is a graph with the horizontal axis representing the real part of the complex impedance at each measurement frequency, and the vertical axis representing the imaginary part with the upper side being the negative side.

In this example, the frequency of the alternating electric current is changed within a range between 20 [kHz] and 125 [kHz]. Under this condition, the solid line represents the results of calculating the reactance component X and the resistance component R on the basis of Formula (1) described above. A star-marked point $P_{Z50}$ is a measurement point at which a resistance component $R_{50}$ and a reactance component $X_{50}$ of the bioimpedance Z are measured using the alternating electric current at a frequency of 50 [kHz].

Generally, with the method for applying alternating electric current to a human body to measure the bioimpedance, the inflection point (extreme point) where the reactance component X of the bioimpedance Z is small is obtained when the measurement frequency of the alternating electric current is at or around 50 [kHz], as shown in Fig. 4.

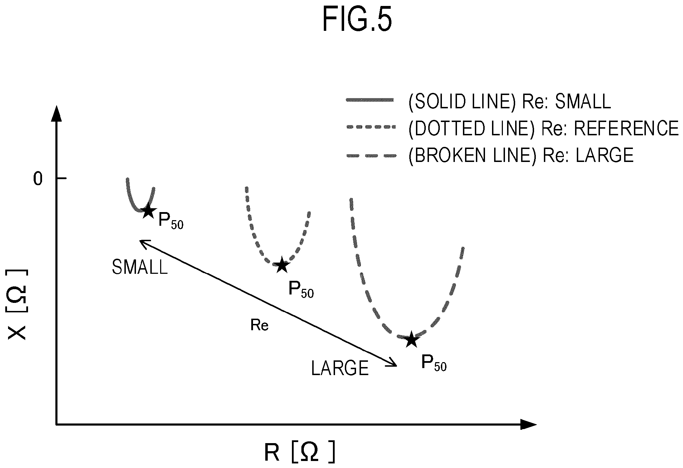

In other words, the minimum reactance component X is obtained when the measurement frequency is at or around 50 [kHz] in a case where the bioimpedance Z of a human body is measured. The minimum point varies depending on the electric resistance Re of the extracellular fluid as shown in the next Fig. 5.

Fig. 5 is a diagram illustrating relationship between a change in the electric resistance Re of the extracellular fluid and a change in the minimum point of the reactance component X.

In Fig. 5, a dotted line represents the Cole-Cole plot obtained with the electric resistance Re of the extracellular fluid being equal to the reference value. Furthermore, a solid line and a broken line represent the Cole-Cole plots obtained with the electric resistance Re of the extracellular fluid being values smaller and larger than the reference value, respectively. Each of these semicircular Cole-Cole plots has a star-marked point $P_{50}$ that is a measurement point of the bioimpedance Z measured with the alternating electric current at the frequency 50 [kHz].

Generally, heavier dehydration leads to a smaller water content of the extracellular fluid, resulting in a larger electric resistance Re of the extracellular fluid. On the other hand, heavier inflammation leads to an increase in the water content of the extracellular fluid, resulting in a smaller electric resistance Re of the extracellular fluid. In this manner, a change in the water content due to the dehydration and inflammation of the measurement part results in a change in the electric resistance Re of the extracellular fluid.

Thus, as shown in Fig. 5, higher severity of the inflammation in the measurement part leads to a smaller electric resistance Re of the extracellular fluid, resulting in a smaller resistance component R and a larger reactance component X of the measurement part.

As described above, a change in the electric resistance Re correlated with the water content of the extracellular fluid results in changes in the minimum point of the reactance component X and in the resistance component R at the minimum point. Thus, when the water content of the extracellular fluid changes due to the inflammation of the measurement part, both reactance component X and resistance component R change. It is a matter of course that a change in the water content of the extracellular fluid involves a change in the bioimpedance including the reactance component X and the resistance component R.

As described above, a change in the electric resistance Re of the extracellular fluid can be captured by capturing the changes in the electric resistance value of at least one of the bioimpedance Z, the reactance component X, and the resistance component R. As a result, the severity of the inflammation in the measurement part can be evaluated as one symptom indicating the condition of the measurement part. Thus, in the present embodiment, the severity of the inflammation in the measurement part can be estimated on the basis the bioimpedance Z of the measurement part correlated with the electric resistance Re of the extracellular fluid.

On the other hand, the electric resistance Re correlated with the water content of the extracellular fluid not only changes in accordance with the inflammation of the measurement part, but also changes in everyday life. For example, the water content of the extracellular fluid largely changes before and after taking a bath, and thus the electric resistance Re of the extracellular fluid also largely changes.

Such a variation in the electric resistance Re of the extracellular fluid in everyday life may offset the change in the electric resistance Re of the extracellular fluid due to the inflammation of the measurement part. Hereinafter, the variation in the electric resistance Re of the extracellular fluid in everyday life will be referred to a diurnal variation and interday variation in the bioimpedance Z.

The diurnal variation and the interday variation are phenomena in which the water content of the extracellular fluid changes with the muscle cell development condition remaining unchanged. The short term change in the body water content is due to the diurnal variation and the interday variation, and the muscle cell development condition almost does not change within approximately three months. Thus, such a change in the body water content is anticipated to be attributable to a movement of the extracellular fluid.

Thus, a short term change in the body weight is not so much affected by a change in the muscular cell but is largely affected by a change in the body water content, meaning that diurnal variation and the interday variation in the bioimpedance Z are highly correlated with a change in the body weight. Thus, in the present embodiment, the evaluation accuracy of the condition at the measurement part is improved with the measurement value of the body weight taken into consideration in addition to the electric resistance value related to the bioimpedance Z for evaluating the inflamed level of the measurement part.

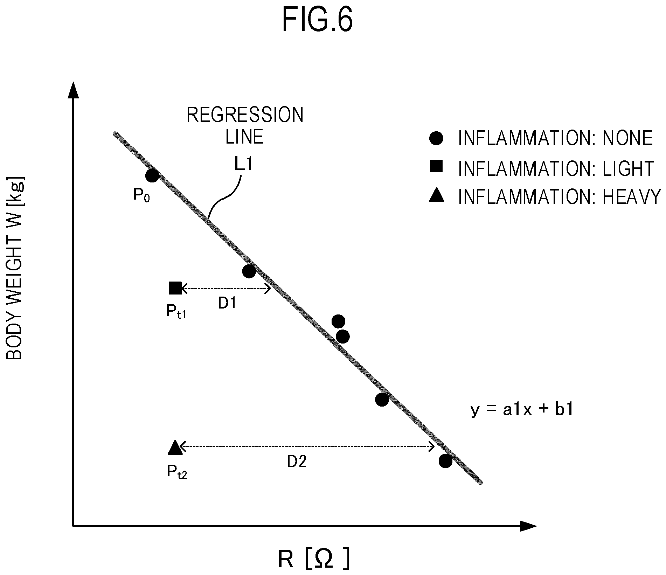

Fig. 6 is a diagram illustrating a relationship between the resistance component R in the bioimpedance Z of the measurement part and the body weight W of the entire user body.

Fig. 6 shows measurement point $P_0$, $P_{t1}$ and $P_{t2}$ that are a plurality of coordinate points identified by measurement values of the body weight W and the resistance component R of the measurement part, and a regression line L1 indicating a correlation between the body weight W of the user and the resistance component R of the body part. Each of the measurement points is measured within a short period of time, specifically, approximately within a month, in an assumed exemplary situation where an athlete is trying to reduce his or her body weight W to a target value within a short period of time.

Circles represent the measurement point Po indicating a normal condition of the measurement part, squares represent the measurement point $P_{t1}$ indicating a lightly inflamed measurement part, and triangles represent measurement point $P_{t2}$ indicating heavily inflamed measurement part.

The regression line L1 is obtained by an approximation method such as least squares method to make the distance between the regression line L1 and each of the measurement points Po (represented by circles) short. The measurement points Po used to obtain the regression line L1 may be past measurement values of the user or group data obtained by gathering measurement values of a specific number of people.

The following Formula (3) represents the quadratic function representing the regression line L1. In this quadratic function, a variable y corresponds to the body weight W of the user and a variable x corresponds to the resistance component R of the measurement part. Coefficients a1 and b1 of the quadratic function represent the regression line L1 and are obtained by an approximation method such as a least squares method or using statistic data and the like.

[Formula 3]

$$y = a_1 x + b_1 \qquad (3)$$

As indicated by the regression line L1, a short term change in the body weight W and a change in the resistance component R of the measurement part are highly correlated with each other. A change in the resistance component R in a daily life is mainly attributable to the movement of the extracellular fluid, and thus is likely to be reflected as a change in the body weight W of the user.

For example, the body weight W of the user changes in accordance with the diurnal variation and the interday variation of the resistance component R and thus the measurement point changes along the regression line L1, under a condition where the content of the extracellular fluid normally changes in the daily life.

Thus, even when the resistance component R changes for a certain amount from the previous value, such a change can be determined as a normal diurnal variation and interday variation in the user in the daily life, as long as the measurement point is close to the regression line L1.

On the other hand, when the measurement part is inflamed due to muscle strain, sprain, or the like, the measurement point identified by each of the measurement values of the body weight W and the resistance component R in the user largely deviates from the regression line L1 as indicated by the square-marked and triangle-marked measurement points $P_{t1}$ and $P_{t2}$.

Such deviation of the measurement point from the regression line L1 is due to the extracellular fluid increased in the measurement part as a result of concentration of bodily fluid in the inflamed measurement part which is unrelated to the diurnal variation and interday variation of the body water content. As a result, reduction of the body weight W of the user may not lead to an increase of the electric resistance Re of the extracellular fluid due to the bodily fluid increasing in the measurement part, and thus the resistance component R may not substantially change at all.

When a measurement part is inflamed, a higher level of the inflammation at the measurement part leads to a larger amount of bodily fluid in the measurement part, resulting in a larger distance between the measurement point of the user and the regression line L1. Thus, as shown in Fig. 6, a distance D2 between the triangle-marked measurement point $P_{t2}$ and the regression line L1 is longer than a distance D1 between the regression line L1 and the square-marked measurement point $P_{t1}$. A change in the measurement point $P_{t1}$ and $P_{t2}$ is assumed to occur in a situation where a part, such as a thigh for example, of a user trying to lose weight is inflamed, and then inflammation worsens.

With the distance between the measurement point of the user and the regression line L1 indicating the correlation between the body weight W of the user and resistance component R thus obtained, the level of inflammation at the measurement part can be accurately evaluated. In this example, the resistance component R is used for capturing a change in the extracellular fluid. Alternatively, the bioimpedance Z or the reactance component X may be used instead of the resistance component R. Also, in such a case, the level of inflammation at the measurement part can be accurately evaluated.

Thus, in the present embodiment, the condition evaluation unit 105 calculates a distance between the measurement point determined by the measurement values of the body weight W of the user and the bioimpedance Z of the measurement part and the regression line L1 used to identify the diurnal variation and interday variation in the bioimpedance Z. Then, the condition evaluation unit 105 evaluates the level of inflammation at the measurement part on the basis of the distance calculated.

The distance between the measurement point of the user and the regression line L1 may be a resistance axial direction distance, a body weight axial direction distance, or a distance from the regression line L1 in a perpendicular direction. The resistance axial direction distance is a reduction amount from the reference value that is the resistance value on the regression line L1 based on the measurement values of the body weight W, to the measurement value of the resistance component R. The body weight axial direction distance is a reduction amount from the reference value that is the body weight value on the regression line L1, to the measurement value of the resistance component R corresponding to the measurement value of the body weight W. The perpendicular direction distance is a distance of a perpendicular line diagonally extending in an upper right direction from the measurement point to the regression line L1.

Regarding the distance between the measurement point of the user and the regression line L1, the resistance axial direction distance tends to most largely change due to an increase in the water content as a result of the inflammation in the measurement part. Thus, the condition evaluation unit 105 according to the present embodiment obtains the distance D in the resistance axial direction as the distance between the measurement point of the user and the regression line L1. As a result, the inflammation of the measurement part can be accurately evaluated.

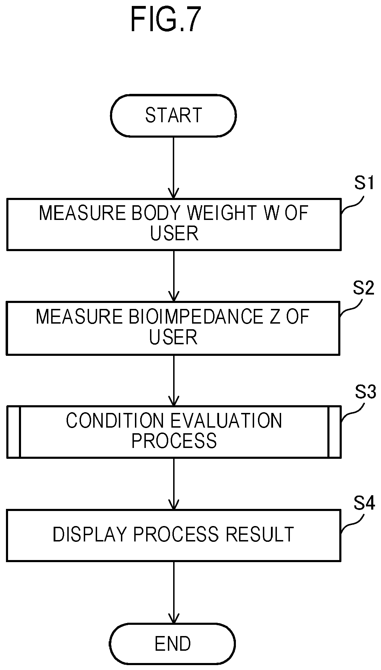

Fig. 7 is a flowchart illustrating an example of a process procedure of a condition evaluation method in the biometric device 10 according to the present embodiment.

In step S1, the body weight measurement unit 102 measures the body weight W of the user to identify the diurnal variation and the interday variation of the bioimpedance Z.

In step S2, the electric resistance measurement unit 103 measures the bioimpedance Z in each part of the user. The electric resistance measurement unit 103 according to the present embodiment calculates the resistance component R of the bioimpedance Z on the basis of Formula (1) described above, for each of the measurement parts.

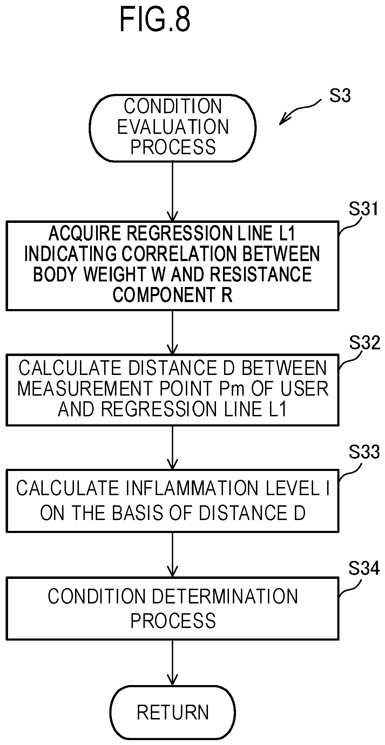

In step S3, the condition evaluation unit 105 executes the condition evaluation process for evaluating the condition of the measurement part based on the body weight W of the user and the bioimpedance Z of the measurement part of the user. The condition evaluation process will be described later with reference to Fig. 8.

In step S4, the display unit 106 displays a result of the condition evaluation process. For example, the display unit 106 displays, for each of the measurement parts, information indicating that the measurement part is inflamed or normal.

When the process in step S4 ends, the control unit 109 terminates a series of processes in the condition evaluation method.

Fig. 8 is a flowchart illustrating an example of a process procedure related to the condition evaluation process executed in step S3. The condition evaluation process is executed for each of the measurement parts in the user.

In step S31, the correlation data holding unit 104 acquires the regression line L1 indicating the relationship between the body weight W and the resistance component R of the measurement part obtained for a specific healthy person with no inflammation, from the communication unit 107 or the storage unit 108, for example. The correlation data holding unit 104 according to the present embodiment acquires the coefficients a1 and b1 of the quadratic function corresponding to the regression line L1, and stores them as the correlation data.

The condition evaluation unit 105 acquires the coefficients a1 and b1 from the correlation data holding unit 104, and calculates the regression line L1 on the basis of the coefficients a1 and b1. Thus, the correlation data holding unit 104 acquires in advance a predetermined regression line indicating the relationship between the bioimpedance Z and the body weight W of a specific person.

In step S32, the condition evaluation unit 105 obtains the distance D between the regression line L1 and the measurement point Pm determined by the measurement values of both of the body weight W of the user and the resistance component R of the measurement part.

The condition evaluation unit 105 according to the present embodiment inputs the measurement value of the body weight W of the user to the variable y of the regression line L1, and calculates an output value of the variable x, as the reference value of the resistance component R. Then, the condition evaluation unit 105 calculates a difference amount between the reference value and the measurement value of the resistance component R, as the distance D between the measurement point Pm and the regression line L1.

In step S33, the condition evaluation unit 105 calculates the inflammation level I indicating the level of inflammation at the measurement part on the basis of the distance D between the regression line L1 and the measurement point Pm of the user. For example, when the distance D is 0, the inflammation level I of the measurement part is 0. The condition evaluation unit 105 determines the inflammation level I to be higher for a larger distance D between the regression line L1 and the measurement point Pm of the user.

In the present embodiment, the correlation data holding unit 104 or the storage unit 108 stores in advance an inflammation table indicating the relationship between the inflammation level I of the measurement part and the difference amount between the reference value and the measurement value for the resistance component R. Upon calculating the difference amount between the reference value and the measurement value for the resistance component R, the condition evaluation unit 105 refers to the inflammation table to calculate the inflammation level I associated with the difference amount thus calculated.

As described above, the condition evaluation unit 105 calculates the inflammation level I of the measurement part by using the body weight W of the user and the bioimpedance Z of the measurement part, as well as the regression line L1 as the regression line indicating the correlation between these two parameters.

In step S34, the condition evaluation unit 105 executes the condition determination process for determining the condition of the measurement part on the basis of the inflammation level I. The condition evaluation unit 105 according to the present embodiment determines the condition of the measurement part on the basis of the inflammation level I, and outputs the result of the determination to the display unit 106.

For example, when the inflammation level I is 0, the condition evaluation unit 105 generates the determination result indicating that the measurement part is normal.

When the process in step S34 ends, the control unit 109 terminates the condition evaluation process that is a subroutine, and returns to the main routine that is the condition evaluation method shown in Fig. 7.

Next, an operation and effect of the first embodiment will be described below.

According to the first embodiment, the biometric device 10 forming the condition evaluation device includes the body weight measurement unit 102 configured to measure the body weight W of the user, and the electric resistance measurement unit 103 configured to measure the bioimpedance Z of a specific part in the user. The biometric device 10 further includes the condition evaluation unit 105 configured to evaluate the condition of the specific part measured by the electric resistance measurement unit 103, on the basis of the body weight W of the user and the bioimpedance Z.

Generally, a variation in body weight W of the user is mainly attributable to a variation of the body water content, and is correlated with the diurnal variation and interday variation in the bioimpedance Z. Thus, in the present embodiment, the condition of a measurement part is evaluated while taking not only the bioimpedance Z of the user but also the body weight W of the user into consideration.

In this way, by taking the body weight W of the user into consideration, it is easy to identify a variation component due to the diurnal variation and interday variation in the measurement value of the bioimpedance Z. Thus, a variation component of the bioimpedance Z due to an abnormal condition of the body part can be accurately extracted. All things considered, the condition of a measurement part in the user can be more accurately evaluated.

The biometric device 10 according to the present embodiment further includes the correlation data holding unit 104 configured to acquire the regression line L1 shown in Fig. 6 as a predetermined regression line indicating the correlation between the body weight W of a person and the bioimpedance Z of a body part. The condition evaluation unit 105 is configured to determine the condition of the measurement part in the user by using the bioimpedance Z and body weight W of the user as well as the predetermined regression line L1.

As a result, with the body weight W of the user applied to the regression line L1 so that the diurnal and interday variations of the bioimpedance Z are identified, the variation component due to the diurnal variation and interday variation can be eliminated from the measurement value of the bioimpedance Z. Thus, whether a measurement part of the user is in a good or a bad condition can be accurately determined.

In the present embodiment, the condition of the measurement part determined by the condition evaluation unit 105 includes a condition involving a change in the body water content, and the condition evaluation unit 105 is configured to calculate the body water content change level indicating the level (degree) of change in the body water content in the measurement part. The body water content change level changes due to an abnormal condition such as inflammation, edema, and muscle atrophy, for example.

Through detection of such a change in the body water content excluding the diurnal variation and interday variation of the user, an abnormal condition of a measurement part involving a change in the body water content can be identified.

In the present embodiment, as shown in Fig. 6, the condition evaluation unit 105 is configured to obtain the distance D between the regression line L1 and the measurement point Pm determined by the bioimpedance Z of the measurement part and the body weight W of the user. And the condition evaluation unit 105 is configured to calculate the inflammation level I indicating the level of inflammation at the measurement part as the body water content change level described above, on the basis of the distance D. The measurement point Pm is a coordinate point plotted in the coordinate system for the body weight W and the bioimpedance Z of the measurement part, on the basis of the acquired values of these parameters.

The level of inflammation at the measurement part is correlated with a change in the bioimpedance Z of the measurement part. Thus, with the distance D between the regression line L1 and the measurement point Pm obtained, a change amount excluding the diurnal and interday variation of the bioimpedance Z of the measurement part can be estimated. Thus, the impact of the diurnal and interday variation related to the bioimpedance Z of the user is reduced, so that the condition evaluation unit 105 can calculate the inflammation level I with a small error.

In the present embodiment, the condition evaluation unit 105 determines the inflammation level I of the measurement part to be larger for a larger reduction amount of the measurement value with respect to the reference value of the bioimpedance Z corresponding to the body weight W of the user on the regression line L1. Thus, the condition evaluation unit 105 can determine the inflammation level I of the measurement part to be higher for a heavier inflammation of the measurement part.

In the present embodiment, the condition evaluation unit 105 determines that the measurement part is inflamed, when the measurement value of the bioimpedance Z of the user, corresponding to the measurement value of the body weight W of the user, is smaller than the reference value on the regression line L1 corresponding to the measurement value of the body weight W of the user.

With determination on the inflammation thus performed with reference to the regression line L1, the diurnal and interday variation of the bioimpedance Z is taken into consideration, so that whether the measurement part is inflamed can be accurately determined.

As described above with reference to Fig. 5, the body water content of the measurement part is also correlated with the electric resistance value of any of the reactance component X and the resistance component R of the bioimpedance Z. Thus, in the present embodiment, the level of inflammation at the measurement part may be evaluated by using the electric resistance value of at least one of the reactance component X and resistance component R instead of the bioimpedance Z. Also in such a case, the level of inflammation at the measurement part, that is, the level of change in the body water content can be accurately evaluated.

(Second embodiment)

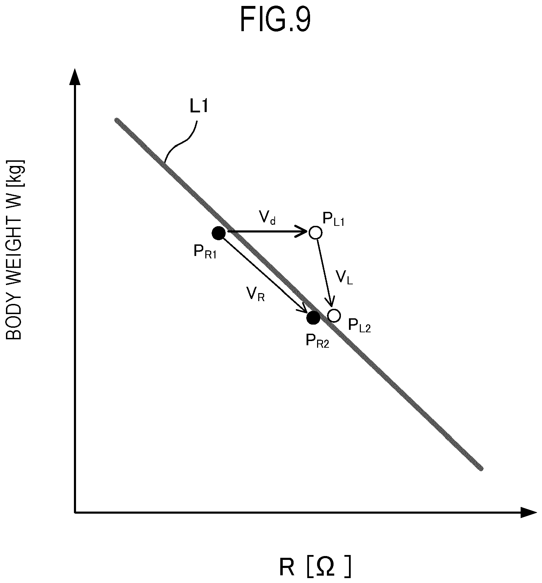

Fig. 9 is a diagram illustrating an example of a method for correcting the inflammation level I according to a second embodiment.

Fig. 9 shows the regression line L1, a previous measurement point $P_{L1}$ and a current measurement point $P_{L2}$ of the left arm, and a previous measurement point $P_{R1}$ and a current measurement point $P_{R2}$ of the right arm. The figure further shows a left arm vector $V_L$ from the previous measurement point $P_{L1}$ to the current measurement point $P_{L2}$ of the left arm, a right arm vector $V_R$ from the previous measurement point $P_{R1}$ to the current measurement point $P_{R2}$ of the right arm.

Generally, the left arm and the right arm are symmetrical with each other, and the muscular development condition is substantially the same between the left arm and the right arm. However, some people may have the development condition being different between the left arm and the right arm.

For example, in a case where a measurement part of user with the muscle on the left arm more atrophied than that on the right arm is evaluated, even when the left arm of the user is inflamed, not only the current measurement point $P_{R2}$ of the right arm but also the current measurement point $P_{L2}$ of the left arm are positioned close to the regression line L1 as shown in Fig. 9.

The current measurement point $P_{L2}$ of the left arm and the current measurement point $P_{R2}$ of the right arm both thus being close to the regression line L1 result in the condition evaluation unit 105 determining that the left arm and the right arm of the user are both normal and thus are not inflamed. This may result in an erroneous determination result indicating that the right arm and the left arm are both normal, even when the left arm of the user has a sign of inflammation.

In view of this, the condition evaluation unit 105 according to the second embodiment corrects the inflammation level I of at least one of the left arm and the right arm, on the basis of the left arm vector $V_L$ and the right arm vector $V_R$.

Specifically, the condition evaluation unit 105 calculates the inflammation level I of the left arm on the basis of the distance D between the regression line L1 and the current measurement point $P_{L2}$ of the left arm, and also calculates the inflammation level I of the right arm on the basis of the distance D between the regression line L1 and the current measurement point $P_{R2}$ of the right arm.

Furthermore, the condition evaluation unit 105 selects one vector which is one of the left arm vector $V_L$ and the right arm vector $V_R$ that is closer to the slope of the regression line L1. In this example, the right arm vector $V_R$ is selected as the one vector.

The condition evaluation unit 105 obtains a difference vector $V_d$ by subtracting the other vector from the selected vector, and corrects the inflammation level I corresponding to the other vector by using the difference vector $V_d$. Specifically, the condition evaluation unit 105 changes the inflammation level I corresponding to the other vector according to values of the direction and the magnitude in the difference vector $V_d$.

A larger difference vector $V_d$ as a result of subtracting the other vector from the selected one vector indicates a large change in the inflammation of the part corresponding to the other vector. The difference vector $V_d$ in a positive direction in the horizontal axis indicates that the inflammation is worsening (or has occurred) in the part corresponding to the other vector. On the other hand, the difference vector $V_d$ in a negative direction in the horizontal axis indicates that the inflammation in the part corresponding to the other vector is healing.

Thus, the condition evaluation unit 105 performs correction to increase the inflammation level I when the difference vector $V_d$ is in the positive direction in the horizontal axis, with the incremented amount increasing as the magnitude of the difference vector $V_d$ increases. Similarly, the condition evaluation unit 105 performs correction to reduce the inflammation level I when the difference vector $V_d$ is in the negative direction in the horizontal axis, with the reduced amount increasing as the magnitude of the difference vector $V_d$ increases.

With the left arm vector $V_L$ and the right arm vector $V_R$ thus used, the inflammation levels I of the measurement parts on the left and the right sides can be more accurately evaluated, even when the muscular development condition of the user is different between the left and the right sides.

In the present embodiment, the inflammation level I of either the left side part or the right side part is corrected. Alternatively, an angle between the regression line L1 and each of the left arm vector $V_L$ and the right arm vector $V_R$ may be calculated, and the inflammation levels I may be corrected on the basis of the angles calculated for each measurement part. Thus, the accuracy of the inflammation levels I on the left and the right measurement parts can be improved.

Next, an operation and effect of the second embodiment will be described below.

In the second embodiment, the electric resistance measurement unit 103 measures the bioimpedance $Z_L$ of the left side part and the bioimpedance $Z_R$ of the right side part, as the bioimpedance Z of the user. Examples of the left side part and the right side part include the left arm and the right arm as well as the left leg and the right leg.

For example, the condition evaluation unit 105 acquires the previous measurement point $P_{L1}$ and the current measurement point $P_{L2}$ for the left arm as the first measurement data and acquires the previous measurement point $P_{R1}$ and the current measurement point $P_{R2}$ of the right arm as the second measurement data, as shown in Fig. 9.

The first measurement data described above is measurement data indicating in time series the left measurement point $P_L$ identified with the body weight W of the user and the bioimpedance Z of the left side part. The second measurement data is measurement data indicating in time series the right measurement point $P_R$ identified with the body weight W of the user and the bioimpedance Z of the right side part.

For example, the first measurement data indicates in time series the measurement values of the body weight W of the user and the bioimpedance Z of the left side part together with the measurement timings. For example, the first measurement data and the second measurement data are stored in the correlation data holding unit 104 or the storage unit 108.

After acquiring the first measurement data and the second measurement data, the condition evaluation unit 105 obtains a vector $V_L$ of a left measurement point $P_L$ on the basis of the first measurement data and obtains a vector $V_R$ of a right measurement point $P_R$ on the basis of the second measurement data. Then, the condition evaluation unit 105 corrects the inflammation level I of at least one of the left side part and the right side part based on the vector $V_L$ of the left measurement point $P_L$ and the vector $V_R$ of the right measurement point $P_R$.

For example, the condition evaluation unit 105 obtains the left arm vector $V_L$ on the basis of the previous measurement point $P_{L1}$ and the current measurement point $P_{L2}$ of the left arm, and obtains the right arm vector $V_R$ on the basis of the previous measurement point $P_{R1}$ and current measurement point $P_{R2}$ of the right arm as shown in Fig. 9. Then, the condition evaluation unit 105 corrects the inflammation level I of the measurement part which is one more deviated from the slope of the regression line L1, on the basis of the left arm vector $V_L$ and the right arm vector $V_R$.

Thus, the transition of the condition of each part is taken into consideration, whereby the inflammation condition of the measurement part can be accurately evaluated even for a user with the muscle condition being different between the right side part and the left side part.

(Third embodiment)

In the embodiment described above, the condition evaluation unit 105 determines that the measurement part of the user is normal when the measurement point Pm1 of the user is positioned near or on the regression line L1. Unfortunately, in a situation where the body weight of the user with a measurement part inflamed is increased due to the overeating of the user, the measurement point Pm1 of the user may be close to the regression line L1 due to the increase in the body weight as a result of the overeating, despite the increase in the water content due to the inflammation of the measurement part.

Specifically, in the embodiment described above, the level of inflammation might fail to be correctly determined with the increase in the water content due to the inflammation occurred in the measurement part of the user regarded as an increase in the water content attributable to the diurnal and interday variation due to an impact of the body weight increase as a result of the overeating of the user.

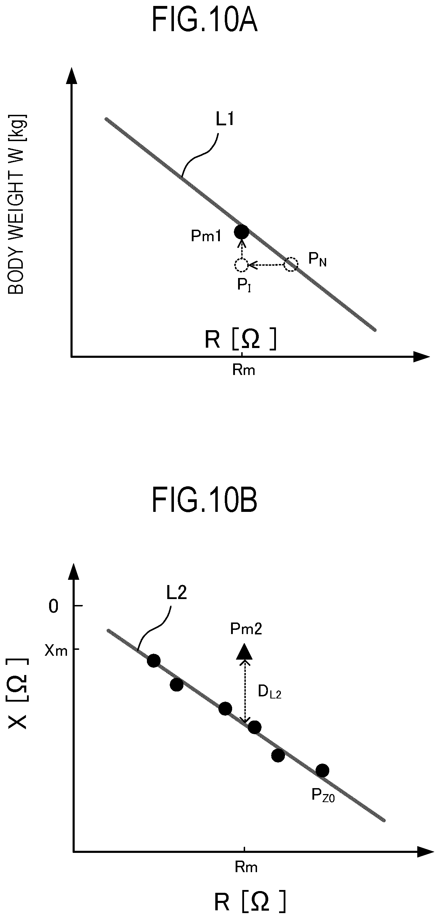

Thus, the condition evaluation unit 105 according to the third embodiment corrects the inflammation level I of the measurement part while taking an increase in the body weight due to the overeating of the user, even when the measurement part of the user is determined to be normal. A method for correcting the inflammation level I of the measurement part will be described with reference to Fig. 10A and Fig. 10B.

Fig. 10A is a diagram illustrating an example of positional relationship between the measurement point Pm1 of the user and the regression line L1.

As shown in Fig. 10A, the measurement point Pm1 of the user is positioned near or on the regression line L1. In such a case, the condition evaluation unit 105 determines that the measurement part of the user is normal.

Still, the measurement point Pm1 of the user is based on the decreased amount of the resistance component R due to the inflammation in the measurement part of the user and the increased amount of the body weight W due to overeating. The measurement point Pm1 of the user will be described below.

To begin with, a normal point $P_N$ indicated by a dotted circle on the regression line L1 is an ideal measurement point of the user who has no inflammation in the measurement part and has not overeaten.

When the measurement part of the user is inflamed, the resistance component R decreases due to the increase in the water content in the measurement part. Thus, the measurement point of the user shifts to an inflammation point $P_I$ in a left direction from the normal point $P_N$ (horizontal axis decreasing direction). In this state, when the user eats too much, the body weight W of the user temporarily increases with the body water content remaining unchanged. As a result, the measurement point of this user shifts to the measurement point Pm1 in an upper direction (vertical axis increasing direction) from the inflammation point $P_I$.

In this manner, even when the water content of the measurement part increases due to the measurement part being inflamed, the measurement point Pm1 might be shifted to a point near or on the regression line L1 due to an increase in the body weight as a result of overeating. This means that a determination result indicating that the measurement part is normal is obtained despite the occurrence of the inflammation of the measurement part.

In view of this, the condition evaluation unit 105 according to the present embodiment checks the determination result for the measurement part, which has been determined to be normal, by using the correlation between the resistance component R and the reactance component X related to the bioimpedance Z of the measurement part.

Fig. 10B is a diagram illustrating an example of positional relationship between a measurement point Pm2 of a user and a regression line L2.

In Fig. 10B, the horizontal axis represents the resistance component R and the vertical axis represents the reactance component X. The measurement point Pm2 is a coordinate point on a complex plane identified by both of a measurement value Xm of the reactance component X and a measurement value Rm of the resistance component R in the measurement part. A measurement point $P_{Z0}$ indicated by a circle is a measurement point as a result of measuring the bioimpedance Z when the measurement part is in a normal state.

The regression line L2 is a predetermined component regression line indicating the relationship between the reactance component X and the resistance component R in the bioimpedance Z of the measurement part. As described above with reference to Fig. 5, a change in the electric resistance Re of the extracellular fluid results in a change in both of the reactance component X and the resistance component R in a fixed direction. Thus, the reactance component X and the resistance component R of the measurement part are correlated with each other as indicated by the regression line L2.

The regression line L2 is obtained by approximation such as a least squares method to make the distance between the regression line L2 and a measurement point $P_{Z0}$ short as in the case of the regression line L1. The measurement point $P_{Z0}$ may be personal data about the user and may be group data including multiple measurement values gathered from a plurality of healthy people without inflammation. For example, the correlation data holding unit 104 or the storage unit 108 stores a coefficient of a quadratic function representing the regression line L2 or the measurement point Pzo used to obtain the regression line L2 as component correlation data.

For example, when the measurement part of the user is normal, the measurement point of the user is plotted near or on the regression line L2. On the other hand, when the measurement part is inflamed, the cell membrane is destroyed and thus the intercellular fluid flows out, resulting in an increase in the amount of extracellular fluid in the measurement part. As a result, the amount of intercellular fluid decreases, and an electric resistance Ri of the intercellular fluid connected to a capacitance Cm of the cell membrane increases, and thus the reactance component X increases as shown in Fig. 3. Thus, the measurement point Pm2 is plotted above the regression line L2 in Fig. 10B when the measurement part is inflamed.

As shown in Fig. 10B, the increased amount of the body water content in the measurement part increases as the measurement value Xm of the reactance component X increases with respect to the reference value on the regression line L2 corresponding to a measurement value Rm of the resistance component R. Thus, a higher level of inflammation at the measurement part results in a large difference between the measurement value of the reactance component X and the reference value on the regression line L2.

Next, an operation of the condition evaluation unit 105 according to the present embodiment will be described.

The condition evaluation unit 105 according to the present embodiment obtains the distance between the measurement point Pm2 of the user and the regression line L2 upon determining that the measurement part to be normal on the basis of the body weight W of the user and the resistance component R of the measurement part as shown in Fig. 10A. Thus, the condition evaluation unit 105 serves as a deviation calculation unit configured to calculate the deviation level on the basis of the distance between the regression line L2 and the measurement point Pm2 determined by the measurement values of both of the reactance component X and the resistance component R. This deviation level increases as the distance between the measurement point Pm2 and the regression line L2 increases for example.

Examples of the distance between the regression line L2 and the measurement point Pm2 of the user include a resistance axial direction distance, a reactance axial direction distance, and a distance in the perpendicular direction with respect to the regression line L2. This resistance axial direction distance is an upward shifting amount to the measurement value Rm from the reference value that is a resistance value on the regression line L2 corresponding to the measurement value Xm of the reactance component X. The reactance axial direction distance is an upward shifting amount to the measurement value Xm of the reactance component X from the reference value that is a reactance value on the regression line L2 corresponding to the measurement value Rm of the resistance component R. The perpendicular direction distance is a distance of the perpendicular diagonally extending in a lower left direction from the measurement point Pm2 to the regression line L2.

The graph shown in Fig. 10B is used to identify the occurrence of the inflammation of the measurement part while taking not only the impact of the resistance component R in Fig. 10A but also taking the impact of the reactance component X into consideration. Thus, the reactance axial direction distance indicating the impact of the reactance component X is preferably used for the distance between the measurement point Pm2 of the user and the regression line L2. Thus, the condition evaluation unit 105 according to the present embodiment obtains a reactance axial direction distance $D_{L2}$ as the distance between the measurement point Pm2 of the user and the regression line L2. Thus, the inflammation of the measurement part can be accurately evaluated.

Then, the condition evaluation unit 105 corrects the inflammation level I, which is substantially 0, on the basis of the distance $D_{L2}$ between the measurement point Pm2 of the user and the regression line L2. For example, the condition evaluation unit 105 performs the correction so that the inflammation level I, which is substantially 0, is increased more for a larger upward shifting amount of the measurement point Pm2 with respect to the regression line L2.

In this manner, the condition evaluation unit 105 according to the present embodiment performs the inflammation evaluation using the correlation between the reactance component X and resistance component R of the measurement part as well as the inflammation evaluation using the correlation between the body weight W of the user and the bioimpedance Z of the measurement part.

With the inflammation evaluation using the correlation between the reactance component X and resistance component R in the measurement part, an impact of the increase in the body weight W due to overeating of the user can be eliminated. Thus, the condition evaluation unit 105 can correctly determine the inflammation of the measurement part even when the bioimpedance Z of a specific part is measured immediately after the overeating by the user. Thus, the condition evaluation unit 105 according to the present embodiment can more accurately evaluate the condition of the measurement part compared with the first embodiment.

In the present embodiment, the regression line L2 is used as the regression line representing the correlation between the reactance component X and the resistance component R. Alternatively, a regression curve expressed by a polynomial may be used.

Next, an operation and effect of the third embodiment will be described.

According to the third embodiment, the correlation data holding unit 104 acquires and holds the predetermined regression line L2 indicating the relationship between the reactance component X and the resistance component R of the measurement part. Furthermore, the electric resistance measurement unit 103 calculates the reactance component X and the resistance component R related to the bioimpedance Z of the user.

Upon determining that the measurement part is normal on the basis of the body weight W of the user and the bioimpedance Z of the measurement part, the condition evaluation unit 105 calculates the deviation level indicating the distance $D_{L2}$ between the regression line L2 and the measurement point Pm2 corresponding to the reactance component X and the resistance component R. The condition evaluation unit 105 corrects the inflammation level I of the measurement part on the basis of the deviation level. The measurement point Pm2 is a coordinate point plotted on a complex plane of the reactance component X and the resistance component R, on the basis of the acquired values of both components.

Thus, the inflammation of the measurement part that has been determined to be normal on the basis of the body weight W of the user and the bioimpedance Z of the measurement part can be accurately evaluated.

(Fourth embodiment)

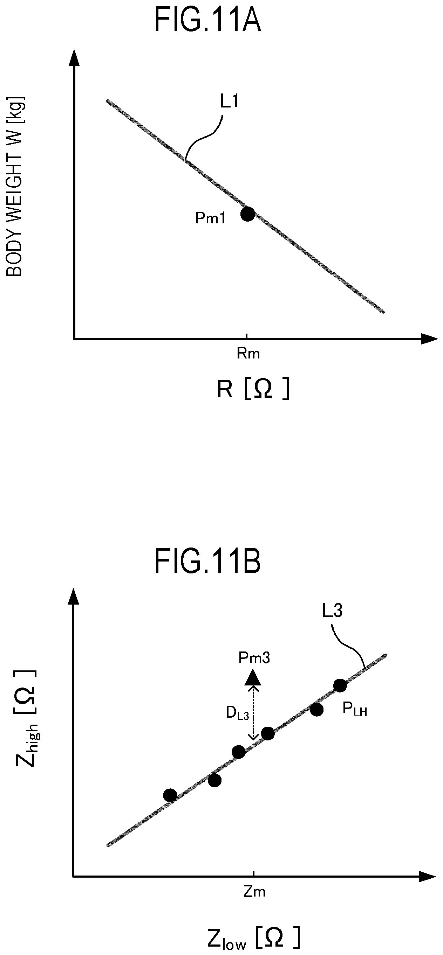

Next, a method for evaluating the condition of a measurement part by the condition evaluation unit 105 according to the fourth embodiment will be described with reference to Fig. 11A and Fig. 11B.

What is shown in Fig. 11A is the same as that in Fig. 10A. Fig. 11B shows an example of positional relationship between a regression line L3 and a measurement point Pm3 of the user. In Fig. 11B, the vertical axis represents a high frequency impedance $Z_{high}$ and the horizontal axis represents a low frequency impedance $Z_{low}$.

The low frequency impedance $Z_{low}$ is the bioimpedance Z measured using a first frequency lower than the reference frequency corresponding to the minimum value of the reactance component X in the Cole-Cole plot. This first frequency is in a range between 20 [kHz] and 50 [kHz]. The low frequency impedance $Z_{low}$ is measured by the electric resistance measurement unit 103.

The high frequency impedance $Z_{high}$ is a bioimpedance Z measured using a second frequency higher than the reference frequency described above. This second frequency is in a range between 50 [kHz] and 250 [kHz]. The high frequency impedance $Z_{high}$ is measured by the electric resistance measurement unit 103.

The measurement point Pm3 is a coordinate point identified by the measurement values of the high frequency impedance $Z_{high}$ and the low frequency impedance $Z_{low}$ on a coordinate system of these impedances.

The regression line L3 is a regression line indicating correlation between the low frequency impedance $Z_{low}$ and the high frequency impedance $Z_{high}$. The low frequency impedance $Z_{low}$ is correlated with the resistance component R. This is because due to the capacitance Cm of the cell membrane, the impact of the electric resistance Ri in the intercellular fluid is small, and the impact of the electric resistance Re in the extracellular fluid is dominant, as shown in Fig. 3.

The high frequency impedance $Z_{high}$ is correlated with the reactance component X. This is because the impact of the electric resistance Ri of the intercellular fluid is larger than that in the case of the low frequency impedance $Z_{low}$ and thus is dominant due to the capacitance Cm of the cell membrane. Thus, the correlation between the low frequency impedance $Z_{low}$ and the high frequency impedance $Z_{high}$ is similar to that between the resistance component R and the reactance component X.

The regression line L3 is obtained by approximation such as a least squares method to make the distance D between the regression line L3 and a measurement point $P_{LH}$ marked with circle short as in the case of the regression line L1. The measurement point $P_{LH}$ may be personal data about the user and may be group data including personal data about a plurality of persons. For example, the correlation data holding unit 104 or the storage unit 108 stores a coefficient of a quadratic function representing the regression line L3 or the measurement point $P_{LH}$ used to obtain the regression line L3 as component correlation data.

As described above, the condition evaluation unit 105 according to the present embodiment acquires the regression line L3 indicating the correlation between the low frequency impedance $Z_{low}$ and the high frequency impedance $Z_{high}$, and obtains the distance D between the regression line L3 and the measurement point Pm3.

Examples of the distance between the regression line L3 and the measurement point Pm3 of the user include low frequency impedance axial direction distance, high frequency impedance axial direction distance, or distance in the perpendicular direction with respect to the regression line L3. This low frequency impedance axial direction distance is a downward shift amount to the measurement value Zm from the low frequency impedance value on the regression line L3 corresponding to the measurement value of the high frequency impedance $Z_{high}$. This high frequency impedance axial direction distance is an upward shift amount to the measurement value Xm of the high frequency impedance $Z_{high}$ from the reference value that is the high frequency impedance value on the regression line L3 corresponding to the measurement value Zm of the low frequency impedance $Z_{low}$. The perpendicular direction distance is a distance of the perpendicular line diagonally extending in an upper right direction from the measurement point Pm3 to the regression line L3.

The graph shown in Fig. 11B is used to identify the occurrence of the inflammation in the measurement part while taking not only the impact of the resistance component R in Fig. 11A but also taking the impact of the reactance component X into consideration. Thus, the high frequency impedance axial direction distance highly correlated with the reactance component X is preferably used for the distance between the measurement point Pm3 and the regression line L3. The condition evaluation unit 105 according to the present embodiment obtains the high frequency impedance axial direction distance $D_{L3}$ as the distance between the regression line L3 and the measurement point Pm3 of the user. Thus, the inflammation of the measurement part can be accurately evaluated.

The condition evaluation unit 105 corrects the inflammation level I of the measurement part on the basis of the distance $D_{L3}$ between the measurement point Pm3 and the regression line L3 upon determining that the measurement part is normal on the basis of the body weight W of the user and the bioimpedance Z of the measurement part.

Next, an operation and effect of the fourth embodiment will be described below.

In the fourth embodiment, the electric resistance measurement unit 103 acquires the low frequency impedance $Z_{low}$ of the user measured by using the first frequency, as the resistance component R of the bioimpedance Z. Furthermore, the electric resistance measurement unit 103 acquires the high frequency impedance $Z_{high}$ of the user measured by using the second frequency higher than the first frequency, as the reactance component X.

Generally, the reactance component X related to the bioimpedance Z is likely to be affected by noise mixed in a measurement cable for each of the electrode portions 3 to 6. In view of this, in the present embodiment, the resistance component R and the reactance component X are respectively replaced by low frequency impedance $Z_{low}$ and the high frequency impedance $Z_{high}$.

Thus, the inflammation level I can be evaluated only using the bioimpedance Z with the guaranteed measurement accuracy in the biometric device 10, whereby the impact of the noise mixed in the measurement cable can be suppressed. Thus, an error of a correction value for correcting the inflammation level I can be reduced.

In the present embodiment, a change in the body water content of the measurement part can be detected as shown in Fig. 11B, by measuring the bioimpedance Z while switching the measurement frequency between the first frequency and the second frequency. Thus, the reactance component X and the resistance component R need not to be calculated. Thus, the calculation load of the electric resistance measurement unit 103 can be reduced from that in the third embodiment, and also the impact of the noise mixed in the measurement cable due to the calculation error of the reactance component X and resistance component R can be eliminated.

A ratio ($Z_{high}/Z_{low}$) of the high frequency impedance $Z_{high}$ to the low frequency impedance $Z_{low}$ is correlated with the resistance component R at the reference frequency. Thus, the horizontal axis in Fig. 11A may represent the ratio ($Z_{high}/Z_{low}$) instead of the resistance component R. Also in this case, the inflammation level I can be accurately evaluated even when the noise is mixed in the measurement cable.

(Fifth embodiment)

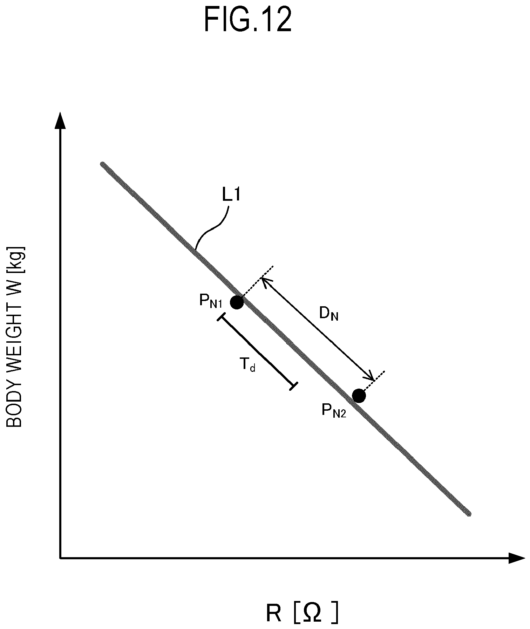

Generally, excessive reduction in the body water content of a user losing weight has a risk of imposing negative impact on the user's health. Thus, in the fifth embodiment, a method for detecting the excessive reduction of the body water content of the user losing weight is described with reference to Fig. 12.

Fig. 12 is a diagram illustrating an example of a method for detecting an excessive reduction of the body water content in the user according to the fifth embodiment. Specifically, the condition evaluation unit 105 according to the present embodiment has a function of determining whether short term dehydration is occurring in the entire body of the user losing weight.

Fig. 12 shows the regression line L1, a previous measurement point $P_{N1}$ and a current measurement point $P_{N2}$ of the measurement part, a distance $D_N$ between the previous measurement point $P_{N1}$ and the current measurement point $P_{N2}$, and a weight loss threshold $T_d$.

As shown in Fig. 12, the current measurement point $P_{N2}$ is near the regression line L1, and thus the condition evaluation unit 105 determines that the measurement part of the user is normal. However, even though the measurement part is not inflamed, the body water content of the user may be excessively reduced. The excessive reduction of the body water content may occur when the user is under excessive weight loss, or when the user was in a sauna for a long period of time.

In view of this, the condition evaluation unit 105 according to the present embodiment determines whether the body weight value at the current measurement point $P_{N2}$ is lower than the body weight value at the previous measurement point $P_{N1}$, and determines whether the distance $D_N$ between the previous measurement point $P_{N1}$ and the current measurement point $P_{N2}$ exceeds the weight loss threshold $T_d$. The condition evaluation unit 105 determines that the body water content of the user has excessively decreased when the body weight value at the current measurement point $P_{N2}$ is lower than the body weight value at the previous measurement point $P_{N1}$, and the distance $D_N$ between the previous measurement point $P_{N1}$ and the current measurement point $P_{N2}$ exceeds the weight loss threshold $T_d$. This weight loss threshold $T_d$ is a threshold used to determine whether the body water content has excessively decreased, and is determined in advance using experimental data, statistical data, and the like.

In the present embodiment, the correlation data holding unit 104 stores the measurement data indicating the previous measurement point $P_{N1}$. The measurement data indicating the previous measurement point $P_{N1}$ includes data in which the previous measurement time, the measurement value of the body weight W of the user, and the measurement value of the resistance component R at the measurement part of the user are associated with each other.

The condition evaluation unit 105 refers to the correlation data holding unit 104 to acquire the previous measurement point $P_{N1}$, and calculates the distance $D_N$ between the previous measurement point $P_{N1}$ and the current measurement point $P_{N2}$. Furthermore, the condition evaluation unit 105 obtains a measurement interval between the measurement timing of the previous measurement point $P_{N1}$ and the measurement timing of the current measurement point $P_{N2}$, and changes the weight loss threshold $T_d$ on the basis of the measurement interval. For example, the condition evaluation unit 105 sets the weight loss threshold $T_d$ to be higher for a longer measurement interval.

The condition evaluation unit 105 determines whether the distance $D_N$ between the previous measurement point $P_{N1}$ and the current measurement point $P_{N2}$ exceeds the weight loss threshold $T_d$. When the distance $D_N$ exceeds the weight loss threshold $T_d$, the condition evaluation unit 105 determines that the body water content of the user has excessively decreased. On the other hand, the condition evaluation unit 105 determines that the user is in a normal state when the distance $D_N$ does not exceed the weight loss threshold $T_d$.

Next, an operation and effect of the fifth embodiment will be describe below.

In the fifth embodiment, the condition evaluation unit 105 determines whether the body water content of the user has excessively decreased on the basis of the distance $D_N$ between the previous measurement point $P_{N1}$ and the current measurement point $P_{N2}$, upon determining that the measurement part is normal on the basis of the current measurement point $P_{N2}$.

Thus, the body water content of the user can be evaluated to have been excessively reduced and thus is in an abnormal condition, even when the measurement part is determined to be normal on the basis of the body weight W and the bioimpedance Z of the measurement part in the user. Thus, unhealthy weight loss of the user can be prevented.

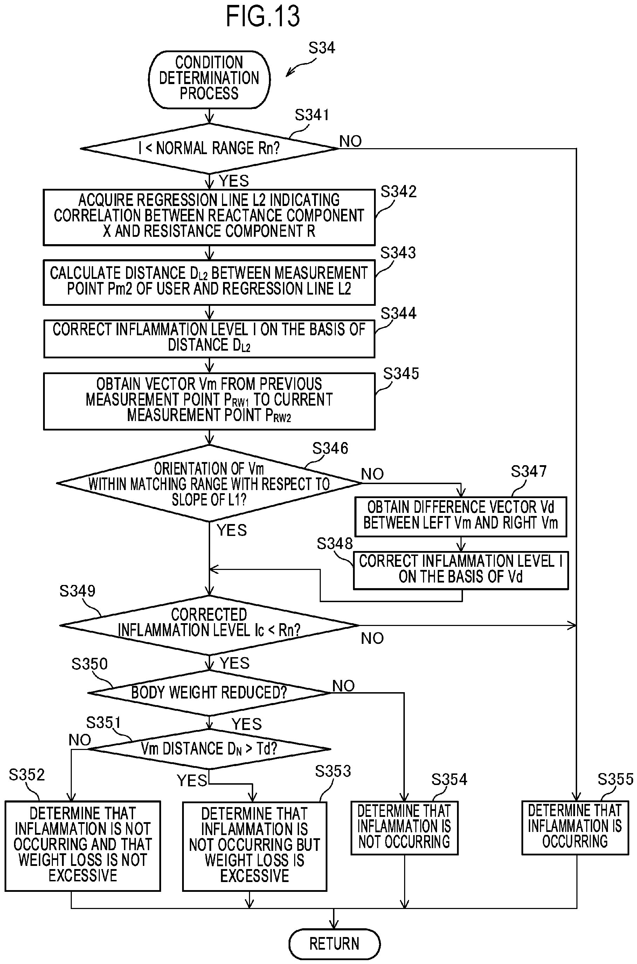

The biometric device 10 may include any one of the third embodiment to the fifth embodiment only, or may include all of the third embodiment to the fifth embodiment. Fig. 13 is a flowchart illustrating an example of an operation of the biometric device 10 including all of the third embodiment to the fifth embodiment.

Fig. 13 is a flowchart illustrating an example of a process procedure of a condition determination process according to the present embodiment. The condition determination process according to the present embodiment is executed in step S34 in Fig. 8 for example.

In step 341, the condition evaluation unit 105 determines whether the inflammation level I obtained on the basis of the distance D between the regression line L1 and the measurement point Pm in the measurement part to be evaluated is within a normal range Rn, as described above with reference to step S33 in Fig. 8.

The normal range Rn described above is 0 or is obtained by adding a calculation error range of the inflammation level I to 0. When the inflammation level I of the measurement part is outside the normal range Rn, the condition evaluation unit 105 proceeds to a process in step S355.

When the inflammation level I of the measurement part is within the normal range Rn, in step S342, the condition evaluation unit 105 acquires the regression line L2 indicating the correlation between the resistance component R and the reactance component X related to the bioimpedance Z as shown in Fig. 10B, from the correlation data holding unit 104.

In step S343, the condition evaluation unit 105 calculates the distance $D_{L2}$ between the regression line L2 and the measurement point Pm2 determined by the measurement values of the resistance component R and the reactance component X in the user.

In step S344, the condition evaluation unit 105 corrects the inflammation level I of the measurement part on the basis of the distance $D_{L2}$ between the measurement point Pm2 and the regression line L2.

In step S345, the condition evaluation unit 105 obtains a measurement vector Vm from a previous measurement point $P_{RW1}$ to a current measurement point $P_{RW2}$ as described above with reference to Fig. 9.

In step S346, the condition evaluation unit 105 determines whether the orientation of the measurement vector Vm is within a matching range with respect to the slope of the regression line L1. This matching range is a value determined while taking a calculation error and the like into consideration. When the orientation of the measurement vector Vm is within a matching range with respect to the slope of the regression line L1, the process proceeds to a process in step S349.

When the orientation of the measurement vector Vm is not within the matching range with respect to the slope of the regression line L1, in step S347, the condition evaluation unit 105 obtains a difference vector $V_d$ between the left and right measurement vectors Vm as shown in Fig. 9. Specifically, the condition evaluation unit 105 obtains the measurement vector Vm of a measurement part on the opposite side of the evaluation target measurement part with the measurement vector Vm having an orientation within the matching range, and obtains the difference vector $V_d$ between the measurement vectors Vm of both measurement parts.

In step S348, the condition evaluation unit 105 corrects the inflammation level I of the measurement part on the basis of the difference vector $V_d$ as described above with reference to Fig. 9.

In step S349, the condition evaluation unit 105 determines whether an inflammation level Ic as a result of correcting inflammation level I at least in step S344 is within the normal range Rn. When the inflammation level Ic corrected is not within the normal range Rn, the condition evaluation unit 105 proceeds to a process in step S355.

When the inflammation level Ic corrected is within the normal range Rn, in step S350, the condition evaluation unit 105 determines whether the current value of the body weight W indicated by the current measurement point $P_{RW2}$ is smaller than the previous value of the body weight W indicated by the previous measurement point $P_{RW1}$. Specifically, the condition evaluation unit 105 determines whether the current value of the body weight W is smaller than the previous value.

When the current value of the body weight W for the user is smaller than the previous value, in step S351, the condition evaluation unit 105 determines whether the distance $D_N$ indicating the magnitude of the measurement vector Vm for the evaluation target exceeds the weight loss threshold Td.

When the distance $D_N$ does not exceed the weight loss threshold Td, in step S352, the condition evaluation unit 105 determines that the measurement part is not inflamed, and that the weight loss is reasonable.

When the distance $D_N$ exceeds the weight loss threshold Td, in step S353, the condition evaluation unit 105 determines that the measurement part is not inflamed, and that the weight loss is excessive as described above with reference to Fig. 12.

When the current value of the body weight W for the user is not smaller than the previous value, in step S354, the condition evaluation unit 105 determines that the measurement part is not inflamed.

When the inflammation level I or Ic is above the normal range Rn in step S341 or S349, the condition evaluation unit 105 determines that the measurement part is inflamed in step S355.

When any of the processes in steps S352 to S355 ends, the condition evaluation unit 105 outputs the determination result to the display unit 106, and returns to the subroutine in Fig. 8.

(Sixth embodiment)

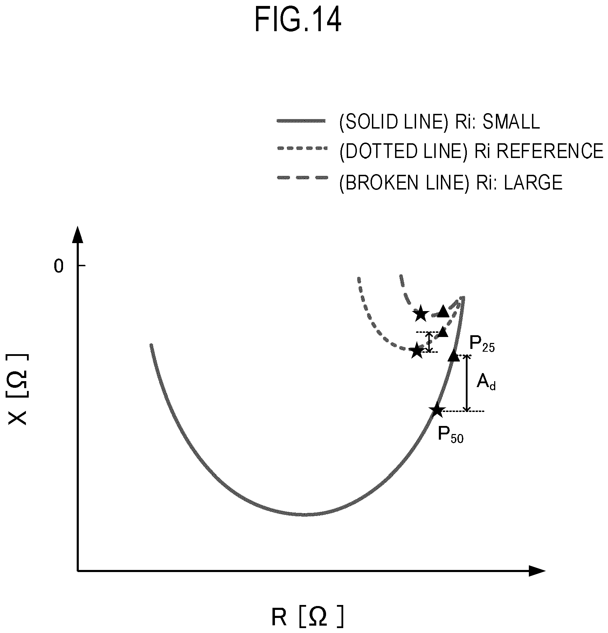

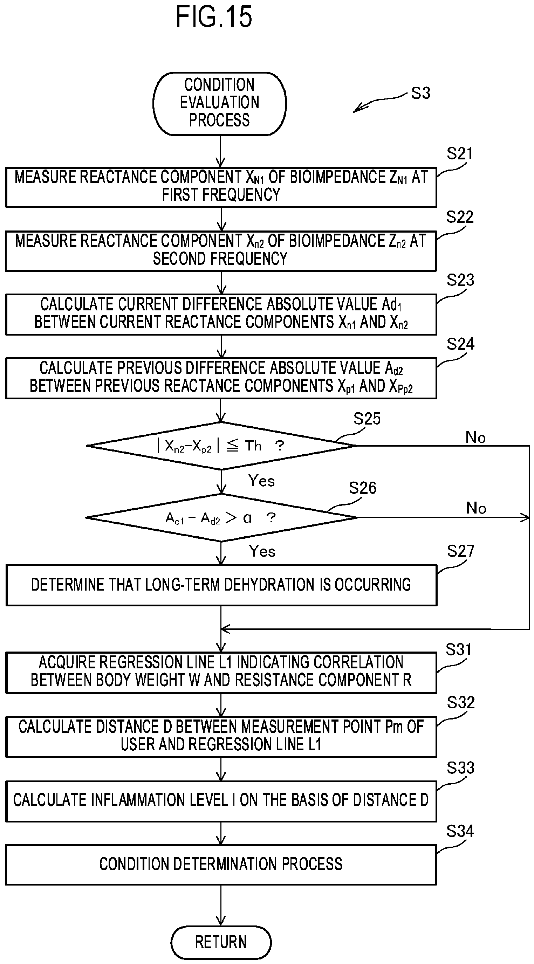

Next, a method for determining whether the entire body of the user is under a long term dehydration according to a sixth embodiment will be described with reference to Figs. 14 and 15.

A long term dehydration of the entire body due to muscle cell atrophy is a possible reason for reducing the electric resistance Ri of the intercellular fluid in a living body. In view of this, a method for capturing a change in the electric resistance Ri of the intercellular fluid will be mainly described in the present embodiment.

Fig. 14 is a diagram illustrating a change in the Cole-Cole plot of the bioimpedance Z due to a change in the intercellular fluid in a case where a change in the extracellular fluid is small.

In Fig. 14, a dotted line corresponds the Cole-Cole plot in a case where the electric resistance Ri of the extracellular fluid is equal to the reference value, and a broken line and a solid line respectively correspond to the Cole-Cole plots in cases where the electric resistance Ri of the extracellular fluid is a value larger and smaller than the reference value, under an assumption that a change in the electric resistance Re of the extracellular fluid is small.

Each Cole-Cole plot of the bioimpedance Z is provided with a star-marked point $P_{50}$ and a triangle-marked point $P_{25}$. The star-marked point $P_{50}$ is a measurement point as a result of measuring the bioimpedance Z with alternating electric current at a frequency 50 [kHz] is applied to the user. The triangle-marked point $P_{25}$ is a measurement point as a result of measuring the bioimpedance Z with alternating electric current at a frequency 25 [kHz] is applied to the user.

As shown in Fig. 14, the reactance component X at the extreme point of the Cole-Cole plot and the resistance component R at the extreme point increase as the electric resistance Ri of the intercellular fluid decreases. On the other hand, the reactance component X at the extreme point of the Cole-Cole plot and the resistance component R at the extreme point decrease as the electric resistance Ri of the intercellular fluid increases.

Similarly, the reactance component X and the resistance component R respectively at the point $P_{25}$ and the point $P_{50}$ increase as the electric resistance Ri of the intercellular fluid decreases, and the reactance component X and the resistance component R at the star-marked points decrease as the electric resistance Ri of the intercellular fluid increases.

Still, if a change in the electric resistance Re of the extracellular fluid is small, an amount of change in the value of the reactance component X measured by using a frequency on the low frequency side in the measurement frequency range in response to a change in the electric resistance Ri of the intercellular fluid is small compared with an amount of change in the value of the reactance component X measured by using a frequency on the high frequency side. Similarly, an amount of change in the value of the resistance component R measured by using a frequency on the low frequency side in response to a change in the electric resistance Ri of the intercellular fluid is small.

Thus, it can be regarded that as long as a change in the electric resistance Re of the extracellular fluid is small, a larger difference absolute value $A_d$ between the reactance components X measured using two frequency points leads to a larger electric resistance Ri of the intercellular fluid. Similarly, it can be regarded that a smaller difference absolute value $A_d$ leads to a smaller electric resistance Ri of the intercellular fluid.

Thus, the condition evaluation unit 105 according to the present embodiment selects at least one of the reactance component X and the resistance component R obtained by using a measurement frequency on the low frequency side that is less affected by a change in the electric resistance Ri of the intercellular fluid, among two measurement frequency points. Then, the condition evaluation unit 105 calculates a time series change amount between the previous value and the current value of the at least one of the components selected, and determines that a change in the electric resistance Re of the extracellular fluid is small when the time series change amount is equal to or smaller than a predetermined threshold.

Furthermore, upon determining that a change in the electric resistance Re of the extracellular fluid is small, the condition evaluation unit 105 determines whether the current value of the difference absolute value $A_d$ between the reactance components X measured using two different frequencies has increased over the previous value thereof. Then, the current value larger than the previous value indicates that the electric resistance Ri of the intercellular fluid has increased. Thus, the condition evaluation unit 105 determines that the entire body of the user is under a long term dehydration.

With a change in the minimum value of the reactance component X in the Cole-Cole plot thus captured, an increase in the electric resistance Ri of the intercellular fluid can be detected, whereby a long-term dehydration of the entire body of the user can be detected.

Fig. 15 is a flowchart illustrating an example of a process procedure of a condition evaluation process according to the present embodiment. The condition evaluation process according to the present embodiment is executed in step S3 in Fig. 7.

In step S21, the electric resistance measurement unit 103 uses the reference frequency, that is, a frequency of 50 [kHz] for example, as the first measurement frequency to measure the reactance component $X_{n1}$ of the measurement part, and records the current value thus measured in the correlation data holding unit 104.

In step S22, the electric resistance measurement unit 103 uses a frequency of 25 [kHz] or 100 [kHz] for example, as a second measurement frequency to measure the reactance component $X_{n2}$ of the measurement part, and records the current value thus measured in the correlation data holding unit 104. In the present embodiment, a frequency lower than the first frequency is used as the second measurement frequency.

In this manner, the electric resistance measurement unit 103 uses a plurality of different frequencies, and measures the reactance component X in the bioimpedance Z at each of the frequencies, for each measurement part.

In step S23, the condition evaluation unit 105 acquires the respective current values $X_{n1}$ and $X_{n2}$ of the reactance component X at the first and the second measurement frequencies from the correlation data holding unit 104, and calculates the current difference absolute value $A_{d1}$ indicating the absolute value of a difference between the current values at the respective measurement frequencies. The current difference absolute value $A_{d1}$ is an absolute value of a difference between the current values $X_{n1}$ and $X_{n2}$ of the reactance components X at two frequency points.

In step S24, the condition evaluation unit 105 acquires the respective previous value $X_{p1}$ and $X_{p2}$ of the reactance component X at the first and the second measurement frequencies from the correlation data holding unit 104, and calculates the previous difference absolute value $A_{d2}$ indicating the absolute value of a difference between the previous values at the measurement frequencies. The previous difference absolute value $A_{d2}$ is an absolute value of a difference between the previous value $X_{p1}$ and $X_{p2}$ of the reactance components X at two frequency points.

In step S25, the condition evaluation unit 105 determines whether the time series change amount of at least one of the reactance component X and the resistance component R on the low frequency side measured by using two different frequency points is equal to or smaller than a predetermined threshold Th. The threshold Th is a value determined in advance on the basis of experiment data or a simulation result, and is configured to determine whether a change in the electric resistance Re of the extracellular fluid is small.

In the present embodiment, the condition evaluation unit 105 determines whether the absolute value of a difference between the previous value $X_{p2}$ and the current value $X_{n2}$ is equal to or smaller than the threshold Th. This difference corresponds to a time series change amount of the low frequency side reactance component X measured by using the second frequency lower than the first frequency. When the time series change amount of the low frequency side reactance component X exceeds the threshold Th, the condition evaluation unit 105 determines that the entire body of the user is not in a long-term dehydrated state, and proceeds to the process in step S31.

When the time series change amount of the low frequency side reactance component X is equal to or smaller than the threshold Th, in step S26, the condition evaluation unit 105 determines whether a difference between the current difference absolute value $A_{d1}$ and the previous difference absolute value $A_{d2}$ is equal to or smaller than a change detection threshold $\alpha$. The change detection threshold $\alpha$ is a threshold for identifying a reduction of the intercellular fluid, and is determined in advance to detect a reduction of the extreme point on the Cole-Cole plot of the bioimpedance Z.

A difference between the current difference absolute value $A_{d1}$ and the previous difference absolute value $A_{d2}$ exceeding the change detection threshold $\alpha$ indicates a possible muscle cell atrophy. Thus, when such a difference is detected in step S27, the condition evaluation unit 105 determines that the entire body of the user is under a long-term dehydration.

The control unit 109 proceeds to a process in step S31 when the process in step S27 ends, when the difference $(A_{d1}-A_{d2})$ is equal to or smaller than the change detection threshold $\alpha$ in step S26, or when the time series change amount $(|X_{n2}-X_{p2}|)$ exceeds the threshold Th in step S25.

In the example described with reference to Fig. 15, the electric resistance measurement unit 103 measures the reactance component X only in steps S21 and S22. Alternatively, the electric resistance measurement unit 103 may measure the resistance component R in addition to the reactance component X.

In such a case, the condition evaluation unit 105 may determine whether the predetermined threshold is exceeded by the time series change amount of the resistance component R on the low frequency side instead of or in addition to the determination on the time series change amount of the reactance component X on the low frequency side in step S24. For example, the condition evaluation unit 105 determines that a change in the electric resistance Re of the extracellular fluid is small, when the time series change amount of the low frequency side reactance component X is equal to or smaller than the threshold Th and when the time series change amount of the low frequency side resistance component R is equal to or smaller than the threshold.

Next, an operation and effect of the sixth embodiment will be described.

In the sixth embodiment, the electric resistance measurement unit 103 uses a plurality of different frequencies, and thus measures the reactance component X in the bioimpedance Z of the user for each of the frequencies. When a change in the extracellular fluid is small, the condition evaluation unit 105 determines whether the entire body is under a long-term dehydration, on the basis of the minimum value of the current reactance component X at each frequency and the minimum value of the previous reactance component X at each frequency.

With a change between the minimum value of the current reactance component X and the minimum value of the previous reactance component X thus detected when a change in the extracellular fluid is small, a change in the electric resistance Ri of the intercellular fluid can be captured as shown in Fig. 14. Thus, the condition of the muscle cell can be identified, whereby whether the entire body is under a long-term dehydration can be determined.

In the present embodiment, the electric resistance measurement unit 103 acquires the reactance components X of the measurement part measured by using the first measurement frequency and the second measurement frequency as the two frequency point, for each of the frequencies. Then, the condition evaluation unit 105 calculates the current difference absolute value $A_{d1}$ and the previous difference absolute value $A_{d2}$ as the minimum values of the reactance components X at the respective frequencies.

As shown in Fig. 14, a larger difference absolute value $A_d$ between the reactance components X at the two frequency points results in a smaller minimum value of the reactance component X at each frequency. Thus, even when the number of measurement frequency points is as small as two, whether the entire body is under a long-term dehydration can be determined. All things considered, the condition of the measurement part can be evaluated in detail while reducing the calculation load of the condition evaluation unit 105.

(Seventh embodiment)

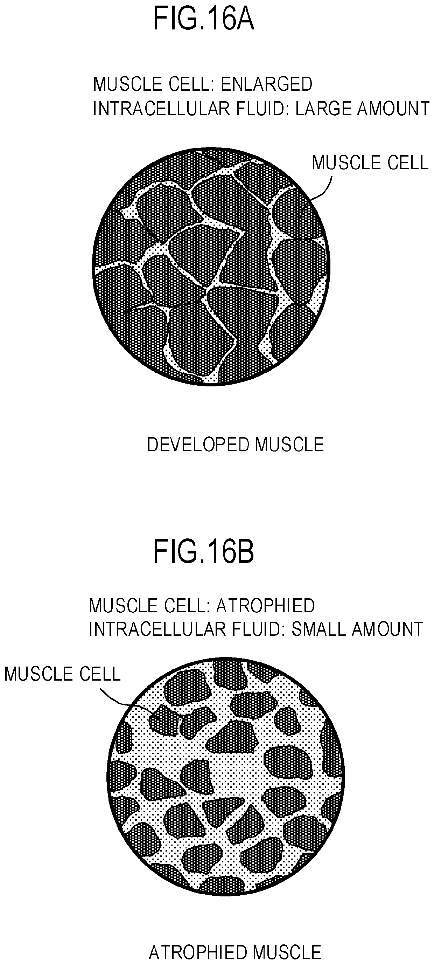

Next, a method for evaluating the condition of muscle cells in the measurement part according to a seventh embodiment will be described with reference to Fig. 16A and Fig. 16B.

Fig. 16A is a diagram illustrating an example of muscle cells in a muscle developed state. As shown in Fig. 16A, in the muscle developed state, the muscle cells are enlarged and thus the amount of intercellular fluid is large.

Fig. 16B is a diagram illustrating an example of muscle cells in a muscle atrophied state. As shown in Fig. 16B, in the muscle atrophied state, the muscle cells are atrophied and thus the amount of intercellular fluid is small.

As described above, the enlargement and atrophy of the muscle cells involves a change in the amount of intercellular fluid. Thus, the development condition of the muscle cells of the user can be evaluated by capturing a change in the electric resistance Ri of the intercellular fluid.

The condition evaluation unit 105 according to the present embodiment detects a change in the extreme point on the Cole-Cole plot of the bioimpedance Z as in the sixth embodiment, to capture a change in the electric resistance Ri of the intercellular fluid.

For example, the condition evaluation unit 105 determines whether the current difference absolute value $A_{d1}$

is larger than the previous difference absolute value $A_{d2}$ as in step S24 in Fig. 15. The condition evaluation unit 105 determines that the muscle in the measurement part of the user is atrophied, when the current difference absolute value $A_{d1}$ is larger than the previous difference absolute value $A_{d2}$, and determines that the muscle in the measurement part of the user is developed when the current difference absolute value $A_{d1}$ is smaller than the previous difference absolute value $A_{d2}$.

It should be noted that the difference absolute value $A_d$ determined with reference to a condition of the muscle of an average person may be used instead of the previous difference absolute value $A_{d2}$. In such a case, the evaluation can be performed through a comparison with an average person.

In this manner, in the seventh embodiment, the development condition of the muscle in the measurement part of the user can be evaluated by capturing a change in the electric resistance Ri of the intercellular fluid.

The determination methods according to the sixth embodiment and the seventh embodiment are similar to each other. Thus, a display content may be switched in accordance with a purpose of the user using the biometric device 10. For example, the operation unit 101 is designed to enable the type of the user to be input. Then, the muscle development condition is displayed when "athlete", "elderly", and "infant" is selected, and whether the entire body is under a long-term dehydration is displayed when "average person" is selected.

While embodiments of the present invention are described, the embodiments described above merely illustrate a part of application examples of the present invention and are not intended to limit the technical range of the present invention to specific structures of the embodiment described above.

The present application claims priority based on Japanese Patent Application No. 2017-254712 filed on December 28, 2017 to the Japan Patent Office, the entire content of which is incorporated herein by reference.

DESCRIPTION OF REFERENCE SIGNS

   10: Biometric device (condition evaluation device)
   102: Body weight measurement unit (body weight acquisition unit)
   103: Electric resistance measurement unit (impedance acquisition unit)
   104: Correlation data holding unit (regression line acquisition unit, component regression line acquisition unit)
   105: Condition evaluation unit (evaluation unit, calculation unit)
   108: Storage unit (program)

**Claims**

1.   A condition evaluation device comprising:

      a body weight acquisition unit configured to acquire a body weight of a user;
      an impedance acquisition unit configured to acquire a bioimpedance of a specific part in the user; and
      an evaluation unit configured to evaluate a condition of the specific part based on the body weight of the user and the bioimpedance of the specific part.

2.   The condition evaluation device according to claim 1, wherein
      the evaluation unit includes a regression line acquisition unit configured to acquire a predetermined regression line indicating correlation between the body weight and the bioimpedance of the body part, and
      the evaluation unit is configured to, by using the body weight of the user, the bioimpedance of the specific part, and the predetermined regression line, determine the condition of the specific part.

3.   The condition evaluation device according to claim 2, wherein
      the condition of the specific part determined by the evaluation unit includes a condition involving a change in a body water content in the specific part, and
      the evaluation unit further includes a calculation unit configured to calculate a body water content change level indicating a level of the change in the body water content in the specific part.

4.   The condition evaluation device according to claim 3, wherein the calculation unit is configured to obtain a distance between the predetermined regression line and a coordinate point determined by the body weight of the user and the bioimpedance of the specific part, and calculate the body water content change level on the basis of the distance.

**5.** The condition evaluation device according to claim 4, wherein
the bioimpedance of the specific part in the user includes a bioimpedance of a left side part in the user and a bioimpedance of a right side part in the user, and
the calculation unit is configured to:

   obtain, on the basis of first acquisition data indicating in time series a left coordinate point determined by the body weight of the user and the bioimpedance of the left side part, a vector of the left coordinate point;
   obtain, on the basis of second acquisition data indicating in time series a right coordinate point determined by the body weight of the user and the bioimpedance of the right side part, a vector of the right coordinate point; and
   correct the body water content change level based on the vector of the left coordinate point and the vector of the right coordinate point.

**6.** The condition evaluation device according to claim 5, wherein
the calculation unit is configured to correct the body water content change level on the basis of a difference vector between the vector of the left coordinate point and the vector of the right coordinate point.

**7.** The condition evaluation device according to any one of claims 3 to 6, wherein
the evaluation unit is configured to determine that the specific part is under a condition involving a change in the body water content, when a bioimpedance of the user corresponding to the body weight of the user is below a value on the predetermined regression line corresponding to the body weight of the user.

**8.** The condition evaluation device according to any one of claims 1 to 7, wherein
the bioimpedance of the specific part includes a value of at least one of a reactance component and a resistance component in the bioimpedance.

**9.** The condition evaluation device according to claim 7 further comprising:

   a component regression line acquisition unit configured to acquire a predetermined component regression line indicating relationship between a reactance component and a resistance component of the bioimpedance of the specific part;
   a component calculation unit configured to calculate values of the reactance component and the resistance component in the bioimpedance of the specific part acquired by the impedance acquisition unit; and
   a deviation calculation unit configured to calculate a deviation level between the predetermined component regression line and a coordinate point determined by the calculated values of the reactance component and the resistance component, wherein
   when the specific part is determined to be normal on the basis of the body water content change level, the evaluation unit is configured to correct the body water content change level on the basis of the deviation level.

**10.** The condition evaluation device according to claim 9, wherein
the impedance acquisition unit is configured to:

   acquire a bioimpedance of the user using a first frequency as the reactance component; and
   acquire a bioimpedance of the user using a second frequency higher than the first frequency as the resistance component.

**11.** The condition evaluation device according to any one of claims 1 to 10, wherein
the impedance acquisition unit is configured to acquire reactance components in the bioimpedance of entire body or the specific part in the user by using a plurality of different frequencies, and
the evaluation unit is configured to determine whether the entire body of the user is under a long-term dehydration on the basis of a minimum value of current reactance components at the different frequencies and a minimum value of previous reactance components at the different frequencies.

**12.** The condition evaluation device according to claim 11, wherein
the impedance acquisition unit is configured to acquire the reactance components using two frequency points, and
the evaluation unit is configured to calculate a difference absolute value between the reactance components at the two frequency points, as the minimum value of the reactance components at the different frequencies.

**13.** A condition evaluation method comprising:

a body weight acquisition step of acquiring a body weight of a user;
an impedance acquisition step of acquiring a bioimpedance of a specific part in the user; and
an evaluation steps of evaluating a condition of the specific part based on the body weight of the user and the bioimpedance of the specific part.

14. A program configured to cause a computer to execute:

a body weight acquisition step of acquiring a body weight of a user;
an impedance acquisition step of acquiring a bioimpedance of a specific part in the user; and
an evaluation steps of evaluating a condition of the specific part based on the body weight of the user and the bioimpedance of the specific part.

# FIG.1

BIOMETRIC DEVICE
10

# FIG.2

OPERATION
UNIT
101

BODY WEIGHT
MEASUREMENT
UNIT
102

ELECTRIC
RESISTANCE
MEASUREMENT
UNIT
103

CORRELATION
DATA HOLDING
UNIT
104

CONTROL
UNIT
109

CONDITION
EVALUATION
UNIT
105

DISPLAY
UNIT
106

COMMUNICATION
UNIT
107

STORAGE
UNIT
108

10

# FIG.3

EQUIVALENT CIRCUIT
100

EXTRACELLULAR FLUID

Re

CELL
MEMBRANE

INTRACELLULAR
FLUID

Ri

Cm

# FIG.4

FIG.5

(SOLID LINE) Re: SMALL
(DOTTED LINE) Re: REFERENCE
(BROKEN LINE) Re: LARGE

# FIG.6

REGRESSION LINE L1

BODY WEIGHT W [kg]

● INFLAMMATION: NONE
■ INFLAMMATION: LIGHT
▲ INFLAMMATION: HEAVY

$P_0$

$P_{t1}$ D1

$P_{t2}$ D2

$y = a1x + b1$

R [Ω]

# FIG.7

```
                    START

                       │
                       ▼
        ┌──────────────────────────────┐   S1
        │   MEASURE BODY WEIGHT W OF    │
        │            USER              │
        └──────────────────────────────┘
                       │
                       ▼
        ┌──────────────────────────────┐   S2
        │   MEASURE BIOIMPEDANCE Z OF   │
        │            USER              │
        └──────────────────────────────┘
                       │
                       ▼
        ┌──────────────────────────────┐   S3
        │     CONDITION EVALUATION      │
        │           PROCESS            │
        └──────────────────────────────┘
                       │
                       ▼
        ┌──────────────────────────────┐   S4
        │     DISPLAY PROCESS RESULT    │
        └──────────────────────────────┘
                       │
                       ▼
                      END
```

# FIG.8

```
       ┌─────────────────┐
       │    CONDITION    │        S3
       │   EVALUATION    │
       │     PROCESS     │
       └─────────────────┘
                │
                ▼
┌───────────────────────────────────┐  S31
│      ACQUIRE REGRESSION LINE L1    │
│  INDICATING CORRELATION BETWEEN    │
│  BODY WEIGHT W AND RESISTANCE      │
│            COMPONENT R             │
└───────────────────────────────────┘
                │
                ▼
┌───────────────────────────────────┐  S32
│   CALCULATE DISTANCE D BETWEEN     │
│ MEASUREMENT POINT Pm OF USER       │
│      AND REGRESSION LINE L1        │
└───────────────────────────────────┘
                │
                ▼
┌───────────────────────────────────┐  S33
│  CALCULATE INFLAMMATION LEVEL I    │
│    ON THE BASIS OF DISTANCE D      │
└───────────────────────────────────┘
                │
                ▼
┌───────────────────────────────────┐  S34
│    CONDITION DETERMINATION         │
│           PROCESS                  │
└───────────────────────────────────┘
                │
                ▼
        ┌─────────────────┐
        │     RETURN      │
        └─────────────────┘
```

# FIG.9

# FIG.10A

# FIG.10B

# FIG.11A

# FIG.11B

## FIG.12

# FIG.13

```
        ┌─────────────────┐      S34
        │   CONDITION     │    ↙
        │  DETERMINATION  │
        │    PROCESS      │
        └─────────────────┘
                │
                ▼                      S341
        ◇─────────────────────────◇  NO
         I < NORMAL RANGE Rn? ─────────────────────────────┐
        ◇─────────────────────────◇                        │
                │ YES                                       │
                ▼                                S342       │
    ┌──────────────────────────────────────┐               │
    │ ACQUIRE REGRESSION LINE L2 INDICATING │               │
    │ CORRELATION BETWEEN REACTANCE COMPONENT│              │
    │  X AND RESISTANCE COMPONENT R          │              │
    └──────────────────────────────────────┘   S343        │
                │                                           │
                ▼                                           │
    ┌──────────────────────────────────────┐               │
    │ CALCULATE DISTANCE D_L2 BETWEEN MEASUREMENT │         │
    │ POINT Pm2 OF USER AND REGRESSION LINE L2    │         │
    └──────────────────────────────────────┘   S344        │
                │                                           │
                ▼                                           │
    ┌──────────────────────────────────────┐               │
    │ CORRECT INFLAMMATION LEVEL I ON THE BASIS OF │        │
    │          DISTANCE D_L2                 │              │
    └──────────────────────────────────────┘               │
                │                                S345        │
                ▼                                           │
    ┌──────────────────────────────────────┐               │
    │ OBTAIN VECTOR Vm FROM PREVIOUS        │               │
    │ MEASUREMENT POINT P_RW1 TO CURRENT    │               │
    │ MEASUREMENT POINT P_RW2               │               │
    └──────────────────────────────────────┘               │
                │                                           │
                ▼              S346                         │
        ◇─────────────────────────◇  NO      S347          │
         ORIENTATION OF Vm                                  │
         WITHIN MATCHING RANGE WITH RESPECT TO ────┐        │
         SLOPE OF L1?                              ▼        │
        ◇─────────────────────────◇   ┌──────────────────┐ │
                │ YES                  │ OBTAIN DIFFERENCE VECTOR Vd │
                │                      │ BETWEEN LEFT Vm AND RIGHT Vm│
                │                      └──────────────────┘ │
                │               S348          │             │
                │              ┌──────────────────┐         │
                │              │ CORRECT INFLAMMATION LEVEL I │
                │              │   ON THE BASIS OF Vd         │
                │              └──────────────────┘          │
    S349        ▼                      │                     │
    ◇─────────────────────────◇ ←──────┘                    │
     CORRECTED                                               │
     INFLAMMATION LEVEL Ic < Rn? ──────────────────────────→│
    ◇─────────────────────────◇   NO                         │
    S350        │ YES                                        │
    ◇─────────────────────────◇  NO                          │
     BODY WEIGHT REDUCED? ──────────┐                        │
    ◇─────────────────────────◇     │                        │
    S351        │ YES               │                        │
    NO ◇─────────────────────────◇  │                        │
        Vm DISTANCE D_N > Td?       │                        │
       ◇─────────────────────────◇  │                        │
        │          YES │            │                        │
```

S352 / S353 / S354 / S355

| DETERMINE THAT INFLAMMATION IS NOT OCCURRING AND THAT WEIGHT LOSS IS NOT EXCESSIVE | DETERMINE THAT INFLAMMATION IS NOT OCCURRING BUT WEIGHT LOSS IS EXCESSIVE | DETERMINE THAT INFLAMMATION IS NOT OCCURRING | DETERMINE THAT INFLAMMATION IS OCCURRING |

```
                        ┌─────────┐
                        │ RETURN  │
                        └─────────┘
```

36

# FIG.14

----- (SOLID LINE) Ri: SMALL
----- (DOTTED LINE) Ri REFERENCE
----- (BROKEN LINE) Ri: LARGE

# FIG.15

CONDITION EVALUATION PROCESS — S3

**S21** MEASURE REACTANCE COMPONENT $X_{N1}$ OF BIOIMPEDANCE $Z_{N1}$ AT FIRST FREQUENCY

**S22** MEASURE REACTANCE COMPONENT $X_{n2}$ OF BIOIMPEDANCE $Z_{n2}$ AT SECOND FREQUENCY

**S23** CALCULATE CURRENT DIFFERENCE ABSOLUTE VALUE $A_{d1}$ BETWEEN CURRENT REACTANCE COMPONENTS $X_{n1}$ AND $X_{n2}$

**S24** CALCULATE PREVIOUS DIFFERENCE ABSOLUTE VALUE $A_{d2}$ BETWEEN PREVIOUS REACTANCE COMPONENTS $X_{p1}$ AND $X_{Pp2}$

**S25** $|X_{n2}-X_{p2}| \leqq Th$ ? — No

Yes

**S26** $A_{d1} - A_{d2} > \alpha$ ? — No

Yes

**S27** DETERMINE THAT LONG-TERM DEHYDRATION IS OCCURRING

**S31** ACQUIRE REGRESSION LINE L1 INDICATING CORRELATION BETWEEN BODY WEIGHT W AND RESISTANCE COMPONENT R

**S32** CALCULATE DISTANCE D BETWEEN MEASUREMENT POINT $P_m$ OF USER AND REGRESSION LINE L1

**S33** CALCULATE INFLAMMATION LEVEL I ON THE BASIS OF DISTANCE D

**S34** CONDITION DETERMINATION PROCESS

RETURN

# FIG.16A

MUSCLE CELL: ENLARGED
INTRACELLULAR FLUID: LARGE AMOUNT

MUSCLE CELL

DEVELOPED MUSCLE

# FIG.16B

MUSCLE CELL: ATROPHIED
INTRACELLULAR FLUID: SMALL AMOUNT

MUSCLE CELL

ATROPHIED MUSCLE

<div style="text-align:center">**INTERNATIONAL SEARCH REPORT**</div>

| International application No. |
|---|
| PCT/JP2018/048511 |

| A. CLASSIFICATION OF SUBJECT MATTER |
|---|
| Int.Cl. A61B5/05(2006.01)i |
| According to International Patent Classification (IPC) or to both national classification and IPC |

| B. FIELDS SEARCHED |
|---|
| Minimum documentation searched (classification system followed by classification symbols) |
| Int.Cl. A61B5/05 |

| Documentation searched other than minimum documentation to the extent that such documents are included in the fields searched | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

| Electronic data base consulted during the international search (name of data base and, where practicable, search terms used) |
|---|
| |

| C. DOCUMENTS CONSIDERED TO BE RELEVANT | | |
|---|---|---|
| Category* | Citation of document, with indication, where appropriate, of the relevant passages | Relevant to claim No. |
| X<br>A | JP 2016-150098 A (TANITA CORP.) 22 August 2016,<br>paragraphs [0019]-[0144], fig. 1-9 (Family: none) | 1, 8, 13-14<br>2-7, 9-12 |
| A | JP 2002-102194 A (TANITA CORP.) 09 April 2002,<br>paragraph [0012], fig. 4 & US 2002/0040195 A1,<br>paragraph [0092], fig. 4 | 1-14 |

☐ Further documents are listed in the continuation of Box C.　　☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 18 January 2019 (18.01.2019) | 19 March 2019 (19.03.2019) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

**EP 3 733 062 A1**

## REFERENCES CITED IN THE DESCRIPTION

**Patent documents cited in the description**

- JP 4422997 B
- JP 2017254712 A